# EUROPÄISCHE PATENTANMELDUNG

(11) **EP 1 897 864 A1**
(43) Veröffentlichungstag der Anmeldung: **12.03.2008**
(21) Anmeldenummer: 06016401.9
(22) Anmeldetag: 07.08.2006
(51) Int. Cl.: C07C 49/747, C07C 49/753, C07C 49/755, C07D 333/32, C07D 295/18, A61K 31/381, A61K 31/495, A61K 31/54, A61P 35/00

(54) **Anthracen-Derivate und deren Verwendung zur Behandlung gutartiger und bösartiger Tumorerkrankungen**

(71) Anmelder: AEterna Zentaris GmbH, 60314 Frankfurt (DE)
(72) Erfinder: Gerlach, Matthias, 63636 Brachttal (DE); Günther, Eckhard, 63477 Maintal (DE); Schmidt, Peter, 61137 Schöneck (DE); Prinz, Helge, 48329 Havixbeck (DE); Böhm, Konrad, 07747 Jena (DE); Unger, Eberhard, 07751 Jena (DE)

(57) **Zusammenfassung**

Die vorliegende Erfindung stellt neue Anthracen-Derivate gemäß der allgemeinen Formel (I) und (II) sowie ausgewählte Anthracen-Derivat-Verbindungen bereit. Ferner stellt die vorliegende Erfindung ein Verfahren zur Herstellung solcher Anthracen-Derivate bereit. Des Weiteren werden diese Anthracen-Derivate enthaltenden pharmazeutischen Formulierungen bereitgestellt. Die bereitgestellten Anthracen-Derivate sind insbesondere geeignet zur Behandlung oder Prophylaxe von durch die Inhibition der Tubulin-Polymerisation und/oder von durch die Inhibition Microtubuli-basierten Motorproteinen behandelbaren physiologischen und/oder pathophysiologischen Zuständen, insbesondere diverser Tumorerkrankungen, eingesetzt zu werden.

## Beschreibung

### Technisches Gebiet

Die Erfindung betrifft neue Anthracen-Derivate zur Behandlung gutartiger und bösartiger Tumorerkrankungen. Diese Verbindungen wirken als Inhibitoren der Tubulin-Polymerisation und/oder als Inhibitoren Microtubuli-basierter Motorproteine, insbesondere Kinesine und Dyneine, und sind geeignet für die Behandlung oder Prophylaxe von durch die Inhibition der Tubulin-Polymerisation und/oder von durch die Inhibition Microtubuli-basierten Motorproteinen behandelbaren physiologischen und/oder pathophysiologischen Zuständen sowie diverser Tumorerkrankungen.

### Stand der Technik

Für die nächsten Jahre wird ein dramatischer Anstieg der Tumorerkrankungen und der Tumor bedingten Todesfälle weltweit erwartet. Im Jahre 2001 waren weltweit etwa 10 Mio. Menschen an Krebs erkrankt und über 6 Mio. Menschen sind an dieser Erkrankung gestorben. Die Entwicklung von Tumoren ist eine fundamentale Erkrankung höherer Organismen im Pflanzen- und im Tierreich sowie beim Menschen. Das allgemein anerkannte Mehrschrittmodell der Krebsentstehung geht davon aus, dass durch Anhäufung von Mutationen in einer einzelnen Zelle deren Proliferations- und Differenzierungsverhalten so geändert wird, dass letztendlich über benigne Zwischenstufen ein maligner Zustand mit Metastasierung erreicht wird. Hinter dem Begriff Krebs oder Tumor verbirgt sich ein Krankheitsbild mit mehr als 200 verschiedenen Einzelerkrankungen. Tumorerkrankungen können gutartig oder bösartig verlaufen. Wichtige Tumoren sind die der Lunge, der Brust, des Magens, des Gebärmutterhalses, der Prostata, des Kopfes und Halses, des Dick- und Enddarmes, der Leber und des Blutsystems. Hinsichtlich des Verlaufs, der Prognose und des Therapieverhaltens gibt es große Unterschiede. Mehr als 90% der erkannten Fälle betreffen solide Tumoren, die insbesondere in fortgeschrittenen Stadien bzw. bei Metastasierung gegenwärtig schwer oder nicht therapierbar sind. Neben der operative Entfernung oder der Bestrahlung ist die Chemotherapie eine wichtige Säule der Krebsbekämpfung. Trotz großer Fortschritte ist es bisher nicht gelungen, Medikamente zu entwickeln, die bei den weitverbreiteten soliden Tumoren eine deutliche Verlängerung der Überlebenszeit oder gar komplette Heilung bewirken. Zusätzlich tritt das Problem der Resistenzentwicklung bei etablierten Tumortherapeutika in der Klinik zunehmend häufiger auf. Es ist deshalb sinnvoll, neue Arzneimittel zur Bekämpfung der Krebserkrankung zu erfinden.

Ein möglicher Ansatz, Krebserkrankungen zu bekämpfen, ist die Hemmung der Zellteilung (Mitose). Eine große Anzahl von anti-mitotisch wirkenden Arzneimitteln hat sich bisher als wirksam in der klinischen Onkologie bewiesen. Beispiele hierfür sind die Alkaloide Vincristine und Vinblastine im Rahmen der Behandlung von Leukämie und Hodgkin's Lymphom sowie Paclitaxel und Docetaxel für die Behandlung von metastasierenden Brustkrebs, Lungen- und Eierstockkarzinomen.

Diese Substanzen zeigen jedoch einige schwere Nebenwirkungen, wie bspw. Neurotoxizität (Quasthoff S et al., J. Neurol. 2002, 249: 9-17), und bewirken auch das Auftreten von tw. multiplen Resistenzen (Gottesman MM et al., Nat. Rev. Cancer 2002, 2: 48-58; Dumontet C et al., J. Clin. Oncol. 1999, 17: 1061-1070; Sangrajrang S et al., Chemotherapy 2000, 46: 327-334).

Ein relativ junger Forschungsbereich beschäftigt sich mit der Untersuchung von Microtubuli-basierten Motorproteinen. Zu diesen zählen vor allem die Kinesine, die in den Organellentransport und in der Mitose involviert sind (Yildiz A et al., Trends Cell Biol. 2005, 15: 112-120). Die Kinesin-Superfamilie ist ausführlich in Miki H et al. (Proc. Natl. Acad. Sci. USA 2001, 98: 7004-7011) charakterisiert worden. Weitere relevante Arbeiten, die sich mit Kinesinen, ihren vielfältigen Funktionen und ersten Kinesin-Inhibitoren beschäftigen, sind: Hirokawa N et al., Exp. Cell Res. 2004, 301: 50-59; Zhu C et al., Mol. Biol. Cell 2005, 16: 3187-3199; Sarli V et al., ChemMedChem 2006, 1: 293-298.

### Darstellung der Erfindung

Die vorliegende Erfindung hat daher die Aufgabe, neue Substanzen bereitzustellen, die zur Behandlung von Krebserkrankungen geeignet sind.

Die erfinderische Aufgabe wurde in einem Aspekt überraschender Weise dadurch gelöst, dass neue Anthracen-Derivate gemäß der allgemeinen Formel **(I)** 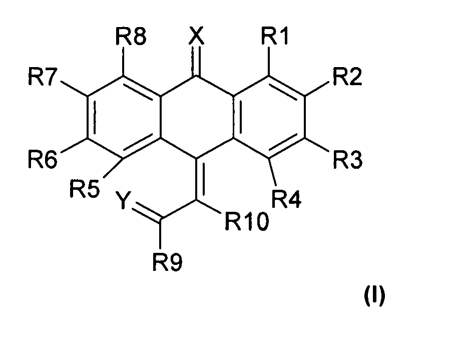 bereitgestellt werden,
worin:
Substituent **X** unabhängig ausgewählt ist aus der Gruppe bestehend aus: "O, S, NR11, N-OR12, geminal geknüpfter Wasserstoff und Hydroxy";
Substituent **Y** unabhängig ausgewählt ist aus der Gruppe bestehend aus: "O, S, NR13, N-OR 14";
Substituenten **R1, R2, R3, R4, R5, R6, R7, R8** unabhängig voneinander unabhängig ausgewählt sind aus der Gruppe bestehend aus: "Wasserstoff, unsubstituiertes oder substituiertes Alkyl, unsubstituiertes oder substituiertes Cycloalkyl, unsubstituiertes oder substituiertes Cycloalkyl-alkyl, unsubstituiertes oder substituiertes Heterocyclyl, unsubstituiertes oder substituiertes Heterocyclyl-alkyl, unsubstituiertes oder substituiertes Aryl, unsubstituiertes oder substituiertes Aryl-alkyl, unsubstituiertes oder substituiertes Heteroaryl, unsubstituiertes oder substituiertes Heteroaryl-alkyl, Amino, Mono-Alkylamino, Di-Alkylamino, Halogen, -F, -Cl, -Br, -I, mit ein oder mehreren Fluoratomen substituiertes Alkyl, Trifluormethyl, Cyano, geradkettiges oder verzweigtes Cyano-alkyl, Carbonyl, Carboxyl, -COOH, Alkoxycarbonyl, Carboxy-alkyl, Alkoxycarbonyl-alkyl, Hydroxy, Alkoxy, Aryl-alkoxy, Benzyloxy, Heteroaryl-alkoxy, Alkoxycarbonylamino, Alkoxycarbonylamino-alkyl";
Substituent **R9** unabhängig ausgewählt ist aus der Gruppe bestehend aus: "unsubstituiertes oder substituiertes Alkyl, unsubstituiertes oder substituiertes Cycloalkyl, unsubstituiertes oder substituiertes Cycloalkyl-alkyl, unsubstituiertes oder substituiertes Heterocyclyl, unsubstituiertes oder substituiertes Heterocyclyl-alkyl, unsubstituiertes oder substituiertes Aryl, unsubstituiertes oder substituiertes Aryl-alkyl, unsubstituiertes oder substituiertes Heteroaryl, unsubstituiertes oder substituiertes Heteroaryl-alkyl, -OR15, -NR16R17";
Substituent **R10** unabhängig ausgewählt ist aus der Gruppe bestehend aus: "Wasserstoff, unsubstituiertes oder substituiertes Alkyl, unsubstituiertes oder substituiertes Aryl, unsubstituiertes oder substituiertes Aryl-alkyl, unsubstituiertes oder substituiertes Heteroaryl, unsubstituiertes oder substituiertes Heteroaryl-alkyl";
Substituenten **R11, R12, R13, R14, R15, R16, R17** unabhängig voneinander unabhängig ausgewählt sind aus der Gruppe bestehend aus: "Wasserstoff, unsubstituiertes oder substituiertes Alkyl, unsubstituiertes oder substituiertes Cycloalkyl, unsubstituiertes oder substituiertes Cycloalkyl-alkyl, unsubstituiertes oder substituiertes Heterocyclyl, unsubstituiertes oder substituiertes Heterocyclyl-alkyl, unsubstituiertes oder substituiertes Aryl, unsubstituiertes oder substituiertes Aryl-alkyl, unsubstituiertes oder substituiertes Heteroaryl, unsubstituiertes oder substituiertes Heteroaryl-alkyl";
mit der Maßgabe, dass die folgenden, unter die allgemeine Formel **(I)** fallenden Verbindungen ausgeschlossen sind:
(i) Verbindung mit der Chemical Abstract Service (CAS) Registry Nr. 262378-52-9 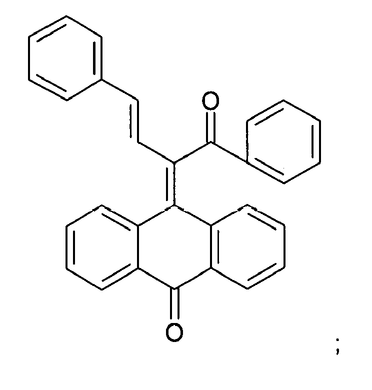
(ii) Verbindung mit der Chemical Abstract Service (CAS) Registry Nr. 121747-08-8 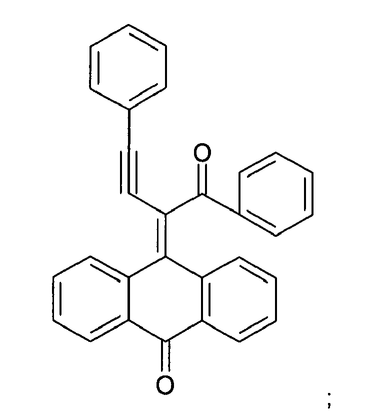
(iii) Verbindung mit der Chemical Abstract Service (CAS) Registry Nr. 63370-49-0 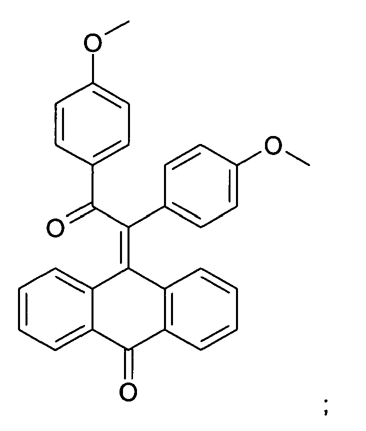
(iv) Verbindung mit der Chemical Abstract Service (CAS) Registry Nr. 19661-62-2 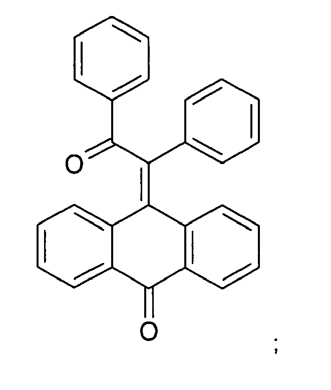
(v) Verbindung mit der Chemical Abstract Service (CAS) Registry Nr. 17665-75-7 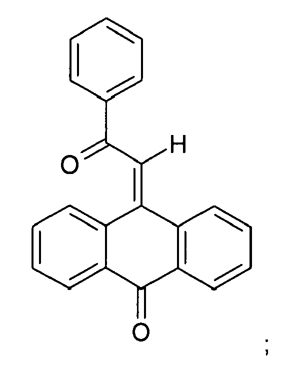
(vi) Verbindung mit der Chemical Abstract Service (CAS) Registry Nr. 83498-19-5

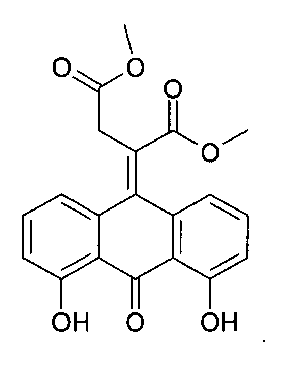

In einer bevorzugten Ausführungsform werden neue Anthracen-Derivat gemäß der allgemeinen Formel **(I)** und gemäß vorangehender obiger Substituenten-Definition bereitgestellt,
worin:
Substituent **X** unabhängig ausgewählt ist aus der Gruppe bestehend aus: "O, geminal geknüpfter Wasserstoff und Hydroxy";
Substituent **Y** unabhängig "O" ist;
Substituenten **R1, R2, R3, R4, R5, R6, R7, R8** unabhängig "Wasserstoff" sind;
Substituent **R9** unabhängig ausgewählt ist aus der Gruppe bestehend aus:
   "unsubstituiertes oder substituiertes Heterocyclyl, unsubstituiertes oder substituiertes Aryl, unsubstituiertes oder substituiertes Heteroaryl";
Substituent **R10** unabhängig "Wasserstoff'' ist.

In einer weiteren bevorzugten Ausführungsform werden neue Anthracen-Derivate gemäß der allgemeinen Formel **(I)** und gemäß der beiden vorangehenden obigen Substituenten-Definitionen bereitgestellt,
worin:
Substituent **R9** unabhängig ausgewählt ist aus der Gruppe bestehend aus: "3,4-Dimethoxy-phenyl; 3,4-Dihydroxy-phenyl; 4-Methoxy-thiophen-2-yl, Thiomorpholin-4-yl; 4-Methoxy-phenyl; 4-(4-Methoxy-phenyl)-piperazin-1-yl, 2-Hydroxy-3,4-dimethoxyphenyl".

In einer weiteren bevorzugten Ausführungsform werden neue Anthracen-Derivate ausgewählt aus der Gruppe bestehend aus:
10-[2-(3,4-Dihydroxy-phenyl)-2-oxo-ethylidene]-10H-anthracen-9-one **(Verbindung 1):** 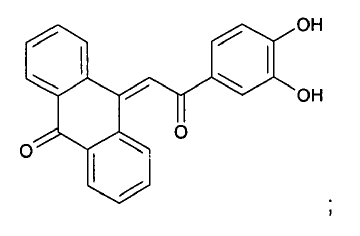
10-[2-(4-Methoxy-thiophen-2-yl)-2-oxo-ethylidene]-10H-anthracen-9-on **(Verbindung 2):** 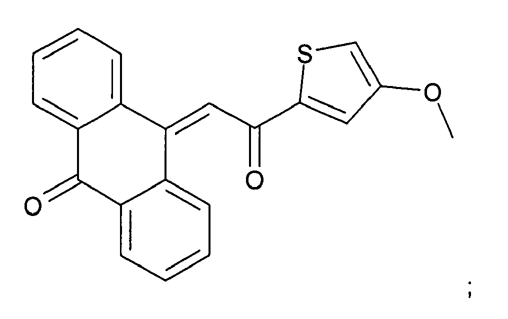
10-(2-Oxo-2-thiomorpholin-4-ylethylidene)-10H-anthracen-9-one **(Verbindung 3):** 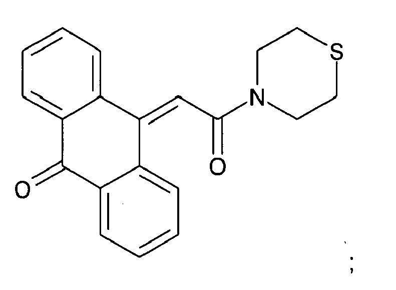
2-(10-Hydroxy-10H-anthracen-9-ylidene)-1-(4-methoxy-phenyl)-ethanone **(Verbindung 4):** 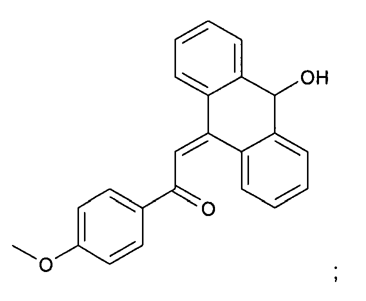
10-{2-[4-(4-Methoxy-phenyl)-piperazin-1-yl]-2-oxo-ethylidene}-10H-anthracen-9-one **(Verbindung 5):** 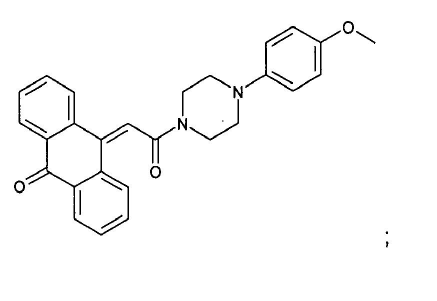
10-[2-(3,4-Dimethoxy-phenyl)-2-oxo-ethyliden]-9(10H)-anthracenon **(Verbindung 6):** 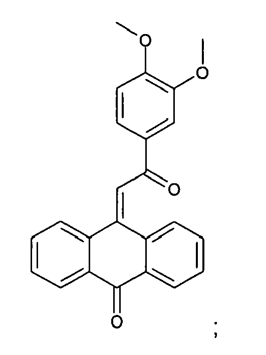
10-[2-(4-Methoxy-phenyl)-2-oxo-ethylidene]-10H-anthracen-9-on **(Verbindung 7):** 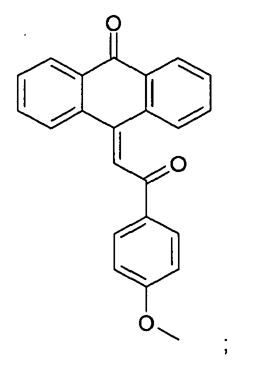
10-[2-(2-Hydroxy-3,4-dimethoxy-phenyl)-2-oxo-ethylidene]-10H-anthracen-9-one **(Verbindung 8)** 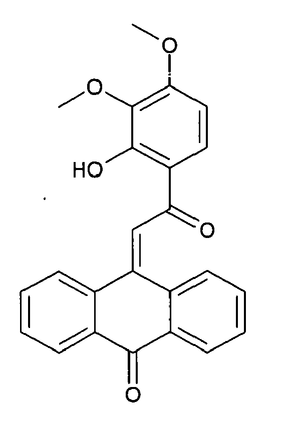 bereitgestellt.

Sollten chemischer Name und chemische Struktur der oben dargestellten Verbindungen aus Fehlergründen nicht korrespondieren, wird - um Unklarheiten vorzubeugen - die chemische Struktur jeder einzelnen Verbindung als maßgeblich für die eindeutige Beschreibung der Verbindung erachtet.

Oben aufgeführte neue Anthracen-Derivate der allgemeinen generischen Formel (I), ihrer beiden bevorzugten generischen Ausführungsformen, die explizit aufgeführten Anthracen-Derivat-Verbindungen **1 bis 8** sowie die Anthracen-Derivate der allgemeinen generischen Formel **(II)** und ihrer beiden bevorzugten generischen Ausführungsformen werden im folgenden gemeinsam als "erfindungsgemäße Verbindungen" bezeichnet.

Die zur Erläuterung der erfindungsgemäßen Verbindungen angegebenen Ausdrücke und Begriffe haben grundsätzlich, sofern in der Beschreibung oder in den Ansprüchen nichts anderes angegeben ist, folgende Bedeutungen:

Der Ausdruck "Alkyl" umfasst im Sinne dieser Erfindung acyclische gesättigte oder ungesättigte Kohlenwasserstoffreste, die verzweigt oder geradkettig sowie unsubstituiert oder ein- oder mehrfach substituiert sein können, mit 1 bis 20 C-Atomen, d.h. C₁-₂₀-Alkanyle, C₂-₂₀-Alkenyle und C₂₋₂₀-Alkinyle, bevorzugt mit 1 bis 8 C-Atomen, d.h. C₁-₈-Alkanyle, C₂-₈-Alkenyle und C₂-₈-Alkinyle. Dabei weisen Alkenyle mindestens eine C-C-Doppelbindung und Alkinyle mindestens eine C-C-Dreifachbindung auf. Bevorzugt ist Alkyl aus der Gruppe ausgewählt, die Methyl, Ethyl, n-Propyl, 2-Propyl, n-Butyl, sec.-Butyl, tert.-Butyl, n-Pentyl, iso-Pentyl, neo-Pentyl, n-Hexyl, 2-Hexyl, n-Octyl, Ethylenyl (Vinyl), Ethinyl, Propenyl (-CH₂CH=CH₂; -CH=CH-CH₃, -C(=CH₂)- CH₃), Propinyl (-CH₂-C≡CH, -C≡C-CH₃), Butenyl, Butinyl, Pentenyl, Pentinyl, Hexenyl, Hexinyl, Octenyl und Octinyl umfasst.

Der Ausdruck "Cycloalkyl" bedeutet für die Zwecke dieser Erfindung cyclische, nicht-aromatische Kohlenwasserstoffe mit 3-12 Kohlenstoffen, die gesättigt oder ungesättigt sein können. Die Bindung an die Verbindungen der allgemeinen Formeln (I) und (II) kann über jedes beliebige und mögliche Ringglied des Cycloalkyl-Restes erfolgen. Der Cycloalkyl-Rest kann auch Teil eines bi- oder polycyclischen Systems sein. Bevorzugt ist Cycloalkyl aus der Gruppe ausgewählt, die Cyclopropyl, Cyclobutyl, Cyclopentyl, Cyclohexyl, Cycloheptyl und Cyclooctyl umfasst.

Der Ausdruck "Heterocyclyl" steht für einen 3-, 4-, 5-, 6-, 7- oder 8-gliedrigen cyclischen organischen Rest, der mindestens 1, ggf. 2, 3, 4 oder 5 Heteroatome enthält,
wobei die Heteroatome gleich oder verschieden sind und der cyclische Rest gesättigt oder ungesättigt, aber nicht aromatisch ist und unsubstituiert oder ein- oder mehrfach substituiert sein kann. Die Bindung an die Verbindungen der allgemeinen Formeln (I) und (II) kann über jedes beliebige und mögliche Ringglied des Heterocyclyl-Restes erfolgen. Der Heterocyclus kann auch Teil eines bi- oder polycyclischen Systems sein. Bevorzugte Heteroatome sind Stickstoff, Sauerstoff und Schwefel. Es ist bevorzugt, dass der Heterocyclyl-Rest ausgewählt ist aus der Gruppe, die Tetrahydrofuryl, Pyrrolidinyl, Imidazolidinyl, Thiazolidinyl, Tetrahydropyranyl, Piperidinyl, Piperazinyl, Morpholinyl und Thiomorpholinyl enthält.

Der Ausdruck "Aryl" bedeutet im Sinne dieser Erfindung aromatische Kohlenwasserstoffe mit 6 bis 14 C-Atomen, u.a. Phenyle, Naphthyle und Anthracenyle. Die Reste können auch mit weiteren gesättigten, (partiell) ungesättigten oder aromatischen Ringsystemen kondensiert sein., wie beispielsweise Tetrahydrofuran, Tetrahydrothiophen, Pyrrolidin, Imidazolidin, Thiazolidin, Tetrahydropyran, Dihydropyran, Piperidin, Furan, Thiophen, Imidazol, Thiazol, Oxazol, Isoxazol. Die Bindung an die Verbindungen der allgemeinen Formeln (I) und (II) kann über jedes beliebige und mögliche Ringglied des Aryl-Restes erfolgen. Jeder Aryl-Rest kann unsubstituiert oder einfach oder mehrfach substituiert vorliegen, wobei die Aryl-Substituenten gleich oder verschieden und in jeder beliebigen und möglichen Position des Aryls sein können.

Der Ausdruck "Heteroaryl" steht für einen 5-, 6- oder 7-gliedrigen cyclischen aromatischen Rest, der mindestens 1, ggf. auch 2, 3, 4 oder 5 Heteroatome enthält, wobei die Heteroatome gleich oder verschieden sind und der Heterocyclus unsubstituiert oder ein- oder mehrfach substituiert sein kann; im Falle der Substitution am Heterocyclus können die Heteroarylsubstituenten gleich oder verschieden sein und in jeder beliebigen und möglichen Position des Heteroaryls sein. Die Bindung an die Verbindungen der allgemeinen Formeln (I) und (II) kann über jedes beliebige und mögliche Ringglied des Heteroaryl-Restes erfolgen. Der Heterocyclus kann auch Teil eines bi- oder polycyclischen Systems sein. Bevorzugte Heteroatome sind Stickstoff, Sauerstoff und Schwefel. Es ist bevorzugt, dass der Heteroaryl-Rest ausgewählt ist aus der Gruppe, die Pyrrolyl, Furyl, Thienyl, Thiazolyl, Oxazolyl, Oxadiazolyl, lsoxazolyl, Pyrazolyl, Imidazolyl, Triazol, Tetrazol, Pyridinyl, Pyrimidinyl, Pyridazinyl, Pyrazinyl, Triazin, Phthalazinyl, Indolyl, Indazolyl, Indolizinyl, Chinolinyl, Isochinolinyl, Chinoxalinyl, Chinazolinyl, Pteridinyl, Carbazolyl, Phenazinyl, Phenoxazinyl, Phenothiazinyl, Acridinyl enthält.

Die Ausdrücke "Cycloalkyl-alkyl", "Heterocyclyl-alkyl", "Aryl-alkyl" oder "Heteroaryl-alkyl" bedeuten für die Zwecke der vorliegenden Erfindung, daß Alkyl, Cycloalkyl, Heterocyclyl, Aryl und Heteroaryl die oben definierten Bedeutungen haben und der Cycloalkyl-, Heterocyclyl-, Aryl- und Heteroaryl-Rest über eine C₁₋₈-Alkyl-Gruppe an die Verbindungen der allgemeinen Formeln (I) und (II) gebunden ist.

Im Zusammenhang mit "Alkyl", "Alkenyl" und "Alkinyl" versteht man unter dem Begriff "substituiert" im Sinne dieser Erfindung die Substitution eines Wasserstoffrestes durch F, CI, Br, I, CN, NH₂, NH-Alkyl, NH-Cycloalkyl, NH-Aryl, NH-Heteroaryl, NH-Aryl-alkyl, NH-Heteroaryl-alkyl, NH-Heterocyclyl, NH-Alkyl-OH, N(Alkyl)₂, N(Aryl-alkyl)₂, N(Heteroaryl-alkyl)₂, N(Heterocyclyl)₂, N(Alkyl-OH)₂, NO, NO₂, SH, S-Alkyl, S-Cycloalkyl, S-Aryl, S-Heteroaryl, S-Aryl-alkyl, S-Heteroaryl-alkyl, S-Heterocyclyl, S-Alkyl-OH, S-Alkyl-SH, S-Alkyl, S-S-Cycloalkyl, S-S-Aryl, S-S-Heteroaryl, S-S-Aryl-Alkyl, S-S-Heteroaryl-alkyl, S-S-Heterocyclyl, SS-Alkyl-OH, S-S-Alkyl-SH, S-S-Alkyl-C(O)-NH-Heterocyclyl, OH, O-Alkyl, O-Cycloalkyl, O-Cycloalkyl-alkyl, O-Aryl, 0-Heteroaryl, O-Aryl-alkyl, O-Heteroaryl-alkyl, O-Heterocyclyl, O-Heterocyclyl-alkyl, O-Alkyl-OH, O-Alkyl-O-Alkyl, O-SO₂-N(Alkyl)₂, O-SO₂-OH O-SO₂-O-Alkyl, O-SO₂-O-Cycloalkyl, O-SO₂-O-Heterocyclylalkyl, O-SO₂-O-Cycloalkyl-alkyl, O-SO₂-O-AlkylHeterocyclyalkyl, O-SO₂-O-Aryl, O-SO₂-O-Heteroaryl, O-SO₂-O-Arylalkyl, O-SO₂-O-Heteroaryl-alkyl, O-SO₂-Alkyl, O-SO₂-Cycloalkyl, O-SO₂-Heterocyclylalkyl, O-SO₂-Cycloalkyl-alkyl, O-SO₂-AlkylHeterocyclylalkyl, O-SO₂-Aryl, O-SO₂-Heteroaryl, O-SO₂-Aryl-alkyl, O-SO₂-Heteroaryl-alkyl, O-C(O)-Alkyl, O-C(O)-Cycloalkyl, O-C(O)-Heterocyclylalkyl, O-C(O)-Cycloalkyl-alkyl, O-C(O)-AlkylHeterocyclyalkyl, O-C(O)-Aryl, O-C(O)-Heteroaryl, O-C(O)-Aryl-alkyl, O-C(O)-Heteroaryl-alkyl, O-C(O)O-Alkyl, O-C(O)O-Cycloalkyl, O-C(O)O-Heterocyclylalkyl, O-C(O)O-Cycloalkyl-alkyl, O-C(O)O-AlkylHeterocyclylalkyl, O-C(O)O-Aryl, O-C(O)O-Heteroaryl, O-C(O)O-Aryl-alkyl, O-C(O)O-Heteroaryl-alkyl, O-C(O)NH-Alkyl, O-C(O)NH-Cycloalkyl, O-C(O)NH-Heterocyclylalkyl, O-C(O)NH-Cycloalkyl-alkyl, O-C(O)NH-AlkylHeterocyclylalkyl, O-C(O)NH-Aryl, O-C(O)NH-Heteroaryl, O-C(O)NH-Aryl-alkyl, O-C(O)NH-Heteroaryl-alkyl, O-C(O)N(Alkyl)₂, O-C(O)N(Cycloalkyl)₂, O-C(O)N(Heterocyclylalkyl)₂, O-C(O)N(Cycloalkyl-alkyl)₂, O-C(O)N(AlkylHeterocyclylalkyl)₂, O-C(O)N(Aryl)₂, O-C(O)N(Heteroaryl)₂, O-C(O)N(Aryl-alkyl)₂, O-C(O)N(Heteroaryl-alkyl)₂, O-P(O)(OH)₂, O-P(O)(O-Metall)₂, O-P(O)(O-Alkyl)₂, O-P(O)(O-Cycloalkyl)₂, O-P(O)(O-Aryl)₂, O-P(O)(O-Heteroaryl)₂, O-P(O)(O-Aryl-alkyl)₂, O-P(O)(O-Heteroaryl-alkyl)₂,O-P(O)(N-Alkyl)₂(N-Alkyl)₂,O-P(O)(N-Cycloalkyl)₂(N-Cycloalkyl)₂,0-P(O)(N-Heterocyclylalkyl)₂(N-Heterocyclyl-alkyl)₂, O-P(O)(N-Aryl)₂(N-Aryl)₂, O-P(O)(N-Heteroaryl)₂(N-Heteroaryl)₂, O-P(O)(N-Aryl-alkyl)₂(N-Aryl-alkyl)₂, O-P(O)(N-Heteroaryl-alkyl)₂(N-Heteroaryl-alkyl)₂, CHO, C(O)-Alkyl, C(S)-Alkyl, C(O)-Aryl, C(S)-Aryl, C(O)-Aryl-alkyl, C(S)-Aryl-alkyl, C(O)-Heterocyclyl, C(O)-Heteroaryl, C(O)-Heteroaryl-alkyl, C(S)-Heterocyclyl, CO₂H, CO₂-Alkyl, CO₂-Cyclyl, CO₂-Heterocyclyl, CO₂-Aryl, CO₂-Heteroaryl, CO₂-Aryl-alkyl, C(O)-NH₂, C(O)NH-Alkyl, C(O)NH-Aryl, C(O)NH-Heterocyclyl, C(O)NH-Alkyl-Heterocyclyl, C(O)N(Alkyl)₂, C(O)N(Aryl-alkyl)₂, C(O)N(Heteroaryl-alkyl)₂, C(O)N(Heterocyclyl)₂, SO-Alkyl, SO₂-Alkyl, SO₂-Aryl, SO₂-Aryl-alkyl, SO₂-Heteroaryl, SO₂-Heteroaryl-alkyl, SO₂NH₂, SO₃H, CF₃, CHO, CHS, Alkyl, Cycloalkyl, Cycloalkyl-alkyl, Aryl, Aryl-alkyl, Heteroaryl, Heteroaryl-alkyl, Heterocyclyl und/oder Heterocyclyl-alkyl, wobei unter mehrfach substituierten Resten solche zu verstehen sind, die entweder an verschiedenen oder an gleichen Atomen mehrfach, z.B. zwei- oder dreifach substituiert sind, beispielsweise dreifach am gleichen C-Atom wie im Falle von CF₃, -CH₂CF₃ oder an verschiedenen Stellen wie im Falle von-CH(OH)-CH=CH-CHCl₂. Die Mehrfachsubstitution kann mit dem gleichen oder verschiedenen Substituenten erfolgen.

In Bezug auf "Aryl", "Aryl-alkyl", "Heteroaryl", Heteroaryl-alkyl", "Heterocyclyl", "Heterocyclyl-alkyl", "Cycloalkyl" sowie "Cycloalkyl-alkyl" versteht man unter dem Begriff "substituiert" im Sinne dieser Erfindung die ein- oder mehrfache Substitution, z.B. zweidrei- oder vierfache Substitution, eines oder mehrerer Wasserstoffatome des Ringsystemes durch F, Cl, Br, I, CN, NH₂, NH-Alkyl, NH-Aryl, NH-Heteroaryl, NH-Aryl-alkyl, NH-Heteroaryl-alkyl, NH-Heterocyclyl, NH-Alkyl-OH, N(Alkyl)₂, NC(O)Alkyl, N(Aryl-alkyl)₂, N(Heteroaryl-alkyl)₂, N(Heterocyclyl)₂, N(Alkyl-OH)₂, NO, NO₂, SH, S-Alkyl, S-Aryl, S-Heteroaryl, S-Aryl-alkyl, S-Heteroaryl-alkyl, S-Heterocyclyl, S-Alkyl-OH, S-Alkyl-SH, OH, O-Alkyl, O-Cycloalkyl, O-Cycloalkyl-alkyl, O-Aryl, O-Heteroaryl, O-Aryl-alkyl, O-Heteroaryl-alkyl, O-Heterocyclyl, O-Heterocyclyl-alkyl, O-Alkyl-OH, O-Alkyl-O-Alkyl, O-SO₂-N(Alkyl)₂, O-SO₂-OH, O-SO₂-O-Alkyl, O-SO₂-O-Cycloalkyl, O-SO₂-O-Heterocyclylalkyl, O-SO₂-O-Cycloalkyl-alkyl, O-SO₂-O-AlkylHeterocydylalkyl, O-SO₂-O-Aryl, O-SO₂-O-Heteroaryl, O-SO₂-O-Aryl-alkyl, O-SO₂-O-Heteroaryl-alkyl, O-SO₂-Alkyl, O-SO₂-Cycloalkyl, O-SO₂-Heterocyclylalkyl, O-SO₂-Cycloalkyl-alkyl, O-SO₂-AlkylHeterocyclylalkyl, O-SO₂-Aryl, O-SO₂-Heteroaryl, O-SO₂-Aryl-alkyl, O-SO₂-Heteroaryl-alkyl, O-C(O)-Alkyl, O-C(O)-Cycloalkyl, O-C(O)-Heterocyclylalkyl, O-C(O)-Cycloalkyl-alkyl, O-C(O)-AlkylHeterocyclylalkyl, O-C(O)-Aryl, O-C(O)-Heteroaryl, O-C(O)-Aryl-alkyl, O-C(O)-Heteroaryl-alkyl, O-C(O)O-Alkyl, O-C(O)O-Cycloalkyl, O-C(O)O-Heterocyclylalkyl, O-C(O)O-Cycloalkyl-alkyl, O-C(O)O-AlkylHeterocyclylalkyl, O-C(O)O-Aryl, O-C(O)O-Heteroaryl, O-C(O)O-Aryl-alkyl, O-C(O)O-Heteroaryl-alkyl, O-C(O)NH-Alkyl, O-C(O)NH-Cycloalkyl, O-C(O)NH-Heterocyclylalkyl, O-C(O)NH-Cycloalkyl-alkyl, O-C(O)NH-AlkylHeterocyclylalkyl, O-C(O)NH-Aryl, O-C(O)NH-Heteroaryl, O-C(O)NH-Aryl-alkyl, O-C(O)NH-Heteroaryl-alkyl, O-C(O)N(Alkyl)₂, O-C(O)N(Cycloalkyl)₂, O-C(O)N(Heterocyclylalkyl)₂, O-C(O)N(Cycloalkyl-alkyl)₂, O-C(O)N(AlkylHeterocyclylalkyl)₂, O-C(O)N(Aryl)₂, O-C(O)N(Heteroaryl)₂, O-C(O)N(Aryl-alkyl)₂, O-C(O)N(Heteroaryl-alkyl)₂, O-P(O)(OH)₂, O-P(O)(O-Metall)₂, O-P(O)(O-Alkyl)₂, O-P(O)(O-Cycloalkyl)₂, O-P(O)(O-Aryl)₂, O-P(O)(O-Heteroaryl)₂, O-P(O)(O-Aryl-alkyl)₂, O-P(O)(O-Heteroaryl-alkyl)₂,O-P(O)(N-Alkyl)₂(N-Alkyl)₂,O-P(O)(N-Cycloalkyl)₂(N-Cycloalkyl)₂,O-P(O)(N-Heterocyclylalkyl)₂(N-Hetero-cycloalkyl)₂, O-P(O)(N-Aryl)₂(N-Aryl)₂, O-P(O)(N-Heteroaryl)₂(N-Heteroaryl)₂, O-P(O)(N-Aryl-alkyl)₂(N-Aryl-alkyl)₂, O-P(O)(N-Heteroaryl-alkyl)₂(N-Heteroaryl-alkyl)₂, CHO, C(O)-Alkyl, C(S)-Alkyl, C(O)-Aryl, C(S)-Aryl, C(O)-Aryl-alkyl, C(S)-Aryl-alkyl, C(O)-Heterocyclyl, C(S)-Heterocyclyl, CO₂H, CO₂-Alkyl, CO₂-Aryl-alkyl, C(O)-NH₂, C(O)NH-Alkyl, C(O)NH-Aryl, C(O)NH-Heterocyclyl, C(O)N(Alkyl)₂, C(O)N(Aryl-alkyl)₂, C(O)N(Heteroaryl-alkyl)₂, C(O)N(Heterocyclyl)₂, SO-Alkyl, SO₂-Alkyl, SO₂-Aryl, SO₂-Aryl-alkyl, SO₂-Heteroaryl, SO₂-Heteroaryl-alkyl, SO₂NH₂, SO₃H, CF₃, CHO, CHS, Alkyl, Cycloalkyl, Cycloalkyl-alkyl, Aryl, Aryl-alkyl, Heteroaryl, Heteroaryl-alkyl, Heterocyclyl und/oder Heterocyclyl-alkyl, wobei unter mehrfach substituierten Resten solche zu verstehen sind, die entweder an verschiedenen oder an gleichen Atomen mehrfach, z.B. zwei- oder dreifach substituiert sind, beispielsweise dreifach am gleichen C-Atom wie im Falle von CF₃, - CH₂CF₃ oder an verschiedenen Stellen wie im Falle von -CH(OH)-CH=CH-CHCl₂. Die Mehrfachsubstitution kann mit dem gleichen oder verschiedenen Substituenten erfolgen.

Die Ausdrücke "Mono-Alkylamino" und "Di-Alkylamino" bedeuten im Rahmen dieser Erfindung, dass ein bzw. zwei Alkylreste gemäß obiger Definition an ein Stickstoffatom gebunden sind. Die Bindung an die Verbindungen der allgemeinen Formel (I) erfolgt über das Stickstoffatom. Beispiele sind Ethylamino, Dimethylamino und Isopropylethylamino.

Der Ausdruck "Cyano-alkyl" bedeutet im Rahmen dieser Erfindung, dass ein Alkylrest gemäß obiger Definition an eine Cyanogruppe gebunden ist. Die Bindung an die Verbindungen der allgemeinen Formeln (I) und (II) erfolgt über die Cyanogruppe. Beispiele sind Methylcyano und n-Propylcyano.

Der Ausdruck "Carbonyl" bedeutet im Rahmen dieser Erfindung, dass ein Alkyl, Cycloalkyl, Cyloalkyl-alkyl, Aryl, Heteroaryl, Aryl-alkyl, Heteroaryl-alkyl, Heterocyclyl und/oder Heterocyclyl-alkyl Rest gemäß obiger Definition an eine -C(O)- Gruppe gebunden ist. Die Bindung an die Verbindungen der allgemeinen Formeln (I) und (II) erfolgt über die -C(O)- Gruppe. Beispiele sind -C(O)-CH₃, -C(O)-CH₂CH₃, -C(O)-isopropyl und -C(O)-tBu (tBu = tert. Butyl).

Der Ausdruck "Carboxyl" bedeutet im Rahmen dieser Erfindung, dass ein Alkyl, Cycloalkyl, Cyloalkyl-alkyl, Aryl, Heteroaryl, Aryl-alkyl, Heteroaryl-alkyl, Heterocyclyl und/oder Heterocyclyl-alkyl Rest gemäß obiger Definitionen an eine -C(O)O- Gruppe gebunden ist. Die Bindung an die Verbindungen der allgemeinen Formeln (I) und (II) erfolgt über die -C(O)O- Gruppe. Beispiele sind -C(O)O-CH₃ und -C(O)O-phenyl.

Der Ausdruck "Carboxy-alkyl" bedeutet im Rahmen dieser Erfindung, dass eine Carboxy Gruppe gemäß obiger Definition an einen Alkylrest gemäß obiger Definition gebunden ist. Die Bindung an die Verbindungen der allgemeinen Formeln (I) und (II) erfolgt über den Alkylrest.

Der Ausdruck "Alkoxy" bedeutet im Rahmen dieser Erfindung, dass ein Alkylrest gemäß obiger Definition an ein Sauerstoffatom gebunden ist. Die Bindung an die Verbindungen der allgemeinen Formeln (I) und (II) erfolgt über das Sauerstoffatom. Beispiele sind Methoxy, Ethoxy und n-Propyloxy.

Der Ausdruck "Alkoxy" in den Begriffen "Alkoxycarbonyl", "Aryl-alkoxy", "Heteroaryl-alkoxy", "Alkoxycarbonylamino", "Alkoxycarbonylamino-alkyl" hat oben definierte Bedeutung. Die Bindung an die Verbindungen der allgemeinen Formeln (I) und (II) erfolgt bei "Aryl-alkoxy" und "Heteroaryl-alkoxy" über das Sauerstoffatom. Beispiele sind Benzyloxy und Indolyloxy.

Bei "Alkoxycarbonyl" erfolgt die Bindung an die Verbindungen der allgemeinen Formeln (I) und (II) über die -C(O)- Gruppe.

Bei "Alkoxycarbonyl-alkyl" erfolgt die Bindung an die Verbindungen der allgemeinen Formeln (I) und (II) über den Alkylrest gemäß oben definierter Bedeutung.

Bei "Alkoxycarbonylamino" erfolgt die Bindung an die Verbindungen der allgemeinen Formeln (I) und (II) über die Aminogruppe; bei "Alkoxycarbonylamino-alkyl" über den Alkylrest gemäß oben definierter Bedeutung.

Der Ausdruck "Metall" umfasst in diesem Sinne dieser Erfindung Metallionen wie Natrium-, Kalium-, Lithium-, Magnesium-, Calcium-, Zink- und Mangan-lonen.

Der Ausdruck "Halogen" umfasst im Sinne dieser Erfindung die Halogenatome Fluor, Chlor, Brom und lod.

Sofern die erfindungsgemäßen Verbindungen mindestens ein Asymmetriezentrum aufweisen, können sie in Form ihrer Racemate, in Form der reinen Enantiomeren und/oder Diastereomeren oder in Form von Mischungen dieser Enantiomeren und/oder Diastereomeren vorliegen und zwar sowohl in Substanz als auch als pharmazeutisch annehmbare Salze dieser Verbindungen. Die Mischungen können in jedem beliebigen Mischungsverhältnis der Stereoisomeren vorliegen.

So lassen sich beispielsweise die erfindungsgemäßen Verbindungen, welche ein oder mehrere Chiralitätszentren aufweisen und die als Racemate auftreten, nach an sich bekannten Methoden in ihre optischen Isomeren, also Enantiomere oder Diastereomere auftrennen. Die Trennung kann durch Säulentrennung an chiralen Phasen oder durch Umkristallisation aus einem optisch aktiven Lösungsmittel oder unter Verwendung einer optisch aktiven Säure oder Base oder durch Derivatisierung mit einem optisch aktiven Reagenz, wie beispielsweise einem optisch aktiven Alkohol, und anschließender Abspaltung des Restes erfolgen.

Die erfindungsgemäßen Verbindungen können in Form ihrer Doppelbindungsisomere als "reine" E- oder Z-Isomere oder in Form von Mischungen dieser Doppelbindungsisomere vorliegen.

Sofern möglich, können die erfindungsgemäßen Verbindungen in Form der Tautomeren vorliegen.

Die erfindungsgemäßen Verbindungen können, falls sie eine ausreichend basische Gruppe, wie zum Beispiel ein primäres, sekundäres oder tertiäres Amin besitzen, mit anorganischen und organischen Säuren in ihre physiologisch verträglichen Salze überführt werden. Vorzugsweise werden die pharmazeutisch annehmbaren Salze der erfindungsgemäßen Verbindungen mit Salzsäure, Bromwasserstoffsäure, Schwefelsäure, Phosphorsäure, Methansulfonsäure, p-Toluolsulfonsäure, Kohlensäure, Ameisensäure, Essigsäure, Trifluoressigsäure, Sulfoessigsäure, Oxalsäure, Malonsäure, Maleinsäure, Bernsteinsäure, Weinsäure, Traubensäure, Äpfelsäure, Embonsäure, Mandelsäure, Fumarsäure, Milchsäure, Citronensäure, Glutaminsäure oder Asparaginsäure gebildet. Bei den gebildeten Salzen handelt es sich u.a. um Hydrochloride, Hydrobromide, Sulfate, Hydrogensulfate, Phosphate, Methansulfonate, Tosylate, Carbonate, Hydrogencarbonate, Formiate, Acetate, Triflate, Sulfoacetate, Oxalate, Malonate, Maleate, Succinate, Tartrate, Malate, Embonate, Mandelate, Fumarate, Lactate, Citrate, Glutaminate und Aspartate. Die Stöchiometrie der gebildeten Salze der erfindungsgemäßen Verbindungen kann dabei ganzzahlige oder nicht ganzzahlige Vielfache von eins betragen.

Die erfindungsgemäßen Verbindungen können, falls sie eine ausreichend saure Gruppe, wie zum Beispiel die Carboxygruppe oder die Phosphorsäuregruppe enthalten, mit anorganischen und organischen Basen in ihre physiologisch verträglichen Salze überführt werden. Als anorganische Basen kommen beispielsweise Natriumhydroxid, Kaliumhydroxid, Calciumhydroxid, als organische Basen Ethanolamin, Diethanolamin, Triethanolamin, Cyclohexylamin, Dibenzylethylendiamin und Lysin in Betracht. Die Stöchiometrie der gebildeten Salze der erfindungsgemäßen Verbindungen kann dabei ganzzahlige oder nicht ganzzahlige Vielfache von eins betragen.

Die erfindungsgemäßen Verbindungen der allgemeinen Formeln (I) und (II) können, falls sie zur Bildung von Koordinationsverbindungen befähigte Gruppen enthalten, mit Übergangsmetallen in Koordinationsverbindungen / Komplexverbindungen überführt werden. Vorzugsweise werden die pharmazeutisch annehmbaren Koordinationsverbindungen / Komplexverbindungen der erfindungsgemäßen Verbindungen gemäß der allgemeinen Formeln (I) und (II) mit Platinsalzen gebildet.

Die Stöchiometrie der gebildeten Koordinationsverbindungen / Komplexverbindungen der erfindungsgemäßen Verbindungen kann dabei ganzzahlige oder nicht ganzzahlige Verhältnisse der erfindungsgemäßen Verbindungen als Liganden zum Übergangsmetall annehmen.

Ebenfalls bevorzugt sind Solvate und insbesondere Hydrate der erfindungsgemäßen Verbindungen, die z. B. durch Kristallisation aus einem Lösungsmittel oder aus wässriger Lösung erhalten werden können. Es können sich dabei ein, zwei, drei oder beliebig viele Solvat- oder Wasser-Moleküle mit den erfindungsgemäßen Verbindungen zu Solvaten und Hydraten verbinden.

Es ist bekannt, dass chemische Substanzen Festkörper ausbilden, die in verschiedenen Ordnungszuständen vorliegen, die man als polymorphe Formen oder Modifikationen bezeichnet. Die verschiedenen Modifikationen einer polymorphen Substanz können sich in ihren physikalischen Eigenschaften stark unterscheiden. Die erfindungsgemäßen Verbindungen können in verschiedenen polymorphen Formen vorliegen, dabei können bestimmte Modifikationen metastabil sein.

Ebenfalls können die erfindungsgemäßen Verbindungen in Form beliebiger Prodrugs wie beispielsweise Ester, Carbonate, Carbamate, Harnstoffe, Amide oder Phosphate vorkommen, bei denen die tatsächlich biologisch aktive Form erst durch Verstoffwechselung freigesetzt wird.

Es ist bekannt, dass chemische Substanzen im Körper zu Metaboliten umgewandelt werden die gegebenenfalls ebenfalls die erwünschte biologische Wirkung - unter Umständen sogar in stärker ausgeprägter Form - hervorrufen können.

Entsprechende Prodrugs und Metabolite der erfindungsgemäßen Verbindungen sind als zur Erfindung gehörig anzusehen.

Mit den erfindungsgemäßen Verbindungen konnte jetzt überraschenderweise eine antiproliferative Aktivität in unterschiedlichen zellulären Tumor-Modellen aufgezeigt werden.

Des weiteren konnte gezeigt werden, dass die erfindungsgemäßen Verbindungen potente Inhibitoren der Tubulin-Polymerisation und/oder Inhibitoren von die Microtubuli-basierten Motorproteinen, bspw. Kinesinen und Dyneinen, insbesondere von mitotischen Kinesinen, darstellen. Kinesine sind ausführlich in Miki H et al. (Proc. Natl. Acad. Sci. USA 2001, 98: 7004-7011) charakterisiert und klassifiziert worden und umfassen bspw. Kinesin-Superfamilie (KIF) Protein, N-1 Kinesine, KIF5A, KIF5B, KIF5C, N-2 Kinesine, Eg5 (KIF11, KSP), BimC (KIF8), N-3 Kinesine, KIF1A, KIF1B, KIF1C, KIF13A, KIF13B, KIF14, K1F16A, K1F16B, N-4 Kinesine, KIF3A, KIF3B, KIF3C, KIF17, N-5 Kinesine, K1F4, KIF4A, KIF4B, KIF21A, KIF21B, N-6 Kinesine, K1F23 (MKLP1, CHO1), KIF20A (Rab6-KIF), K1F20B (KIpMPP1), MKLP2, N-7 Kinesine, KIF10 (CENP-E), N-8 Kinesine, KIF18A, K1F18B, K1F22 (Kid), KIF19A, KIF19B, N-9 Kinesine, K1F12, N-10 Kinesine, KIF15 (Hklp2), N-11 Kinesine, KIF24, K1F25 (KNSL3), K1F26A, K1F26B, M-Kinesine, KIF2A (K1F2), KIF2B, KIF2C (MCAK), C-1 Kinesine, KIFC1, C-2 Kinesine, KIFC2, KIFC3, K1F6, K1F7 und KIF9.

Der überraschende duale Wirkmechanismus macht die erfindungsgemäßen Verbindungen besonders geeignet als Arzneimittel zur Behandlung von gut- und bösartigen Tumorerkrankungen am Menschen und Tieren. Sowohl das Tubulin-Gerüst als auch die Motorproteine spielen eine wichtige Rolle bei der Mitose, einer spezifischen Phase des Zellzyklus. Durch die vorteilhafte Hemmung eines oder beider Targets wird die korrekte Ausbildung der mitotischen Spindel und damit die Aufteilung des genetischen Materials auf die Tochterzellen verhindert. Die unkontrolliert sich teilende Tumorzelle wird im Zellzyklus arretiert. Dies führt in Folge zu einem Absterben der Tumorzelle durch den programmierten Zelltod (Apoptose).

Dadurch ist eine effektive Bekämpfung des Tumors möglich und mittels der erfindungsgemäßen Verbindungen können (multiple) Resistenzen gegenüber bekannten Tumortherapeutika überwunden werden.

Des Weiteren zeigen die erfindungsgemäßen Verbindungen aufgrund ihrer Spezifität und/oder des dualen Wirkmechanismus vorteilhafterweise keine schweren Nebenwirkungen, wie bspw. Neurotoxizität.

Die erfinderische Aufgabe wurde in einem weiteren Aspekt überraschender Weise dadurch gelöst, dass ein Verfahren zur Herstellung der erfindungsgemäßen Verbindungen bereitgestellt wird.

Die erfindungsgemäßen Verbindungen können im Rahmen dieser Erfindung allen bekannten Säugetieren, insbesondere dem Menschen, zur Behandlung und/oder Prophylaxe verabreicht werden.

In einer anderen bevorzugten Ausführungsform werden die erfindungsgemäßen Verbindungen bereitgestellt zu den oben dargestellten Verwendungen, wobei das Säugetier ausgewählt ist aus der Gruppe bestehend aus: "Mensch, Nutztier, Vieh, Haustier, Rind, Kuh, Schaf, Schwein, Ziege, Pferd, Pony, Esel, Maulesel, Maultier, Hase, Kaninchen, Katze, Hund, Meerschweinchen, Hamster, Ratte, Maus" und bevorzugt ein Mensch ist.

Die erfinderische Aufgabe wurde in einem weiteren Aspekt überraschender Weise dadurch gelöst, dass Anthracen-Derivate gemäß der allgemeinen Formel **(II)** 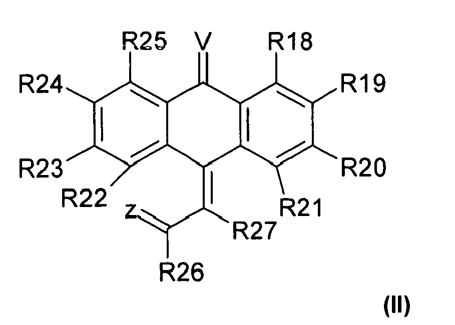 bereitgestellt werden,
worin:
Substituent **V** unabhängig ausgewählt ist aus der Gruppe bestehend aus: "O, S, NR28, N-OR29, geminal geknüpfter Wasserstoff und Hydroxy;
Substituent **Z** unabhängig ausgewählt ist aus der Gruppe bestehend aus: "O, S, NR30, N-OR31";
Substituenten **R18, R19, R20, R21, R22, R23, R24, R25** unabhängig voneinander unabhängig ausgewählt sind aus der Gruppe bestehend aus: "Wasserstoff, unsubstituiertes oder substituiertes Alkyl, unsubstituiertes oder substituiertes Cycloalkyl, unsubstituiertes oder substituiertes Cycloalkyl-alkyl, unsubstituiertes oder substituiertes Heterocyclyl, unsubstituiertes oder substituiertes Heterocyclyl-alkyl, unsubstituiertes oder substituiertes Aryl, unsubstituiertes oder substituiertes Aryl-alkyl, unsubstituiertes oder substituiertes Heteroaryl, unsubstituiertes oder substituiertes Heteroaryl-alkyl, Amino, Mono-Alkylamino, Di-Alkylamino, Halogen, -F, -Cl, -Br,-I, mit ein oder mehreren Fluoratomen substituiertes Alkyl, Trifluormethyl, Cyano, geradkettiges oder verzweigtes Cyano-alkyl, Carbonyl, Carboxyl, -COOH, Alkoxycarbonyl, Carboxy-alkyl, Alkoxycarbonyl-alkyl, Hydroxy, Alkoxy, Aryl-alkoxy, Benzyloxy, Heteroaryl-alkoxy, Alkoxycarbonylamino, Alkoxycarbonylamino-alkyl";
Substituent **R26** unabhängig ausgewählt ist aus der Gruppe bestehend aus: "unsubstituiertes oder substituiertes Alkyl, unsubstituiertes oder substituiertes Cycloalkyl, unsubstituiertes oder substituiertes Cycloalkyl-alkyl, unsubstituiertes oder substituiertes Heterocyclyl, unsubstituiertes oder substituiertes Heterocyclyl-alkyl, unsubstituiertes oder substituiertes Aryl, unsubstituiertes oder substituiertes Aryl-alkyl, unsubstituiertes oder substituiertes Heteroaryl, unsubstituiertes oder substituiertes Heteroaryl-alkyl, -OR32, -NR33R34";
Substituent **R27** unabhängig ausgewählt ist aus der Gruppe bestehend aus: "Wasserstoff, unsubstituiertes oder substituiertes Alkyl, unsubstituiertes oder substituiertes Aryl, unsubstituiertes oder substituiertes Aryl-alkyl, unsubstituiertes oder substituiertes Heteroaryl, unsubstituiertes oder substituiertes Heteroaryl-alkyl";
Substituenten **R28, R29, R30, R31, R32, R33, R34** unabhängig voneinander unabhängig ausgewählt sind aus der Gruppe bestehend aus: "Wasserstoff, unsubstituiertes oder substituiertes Alkyl, unsubstituiertes oder substituiertes Cycloalkyl, unsubstituiertes oder substituiertes Cycloalkyl-alkyl, unsubstituiertes oder substituiertes Heterocyclyl, unsubstituiertes oder substituiertes Heterocyclyl-alkyl, unsubstituiertes oder substituiertes Aryl, unsubstituiertes oder substituiertes Aryl-alkyl, unsubstituiertes oder substituiertes Heteroaryl, unsubstituiertes oder substituiertes Heteroaryl-alkyl";
die zur Herstellung eines Arzneimittels verwendet werden können.

In einer bevorzugten Ausführungsform werden Anthracen-Derivate gemäß der allgemeinen Formel (**II**) wie eben dargestellt bereitgestellt, worin:
Substituent **V** unabhängig ausgewählt ist aus der Gruppe bestehend aus: "O, geminal geknüpfter Wasserstoff und Hydroxy";
Substituent **Z** unabhängig "O" ist;
Substituenten **R18, R19, R20, R21, R22, R23, R24, R25** unabhängig "Wasserstoff" sind;
Substituent **R26** unabhängig ausgewählt ist aus der Gruppe bestehend aus:
   "unsubstituiertes oder substituiertes Heterocyclyl, unsubstituiertes oder substituiertes Aryl, unsubstituiertes oder substituiertes Heteroaryl";
   Substituent **R27** unabhängig "Wasserstoff" ist;
die zur Herstellung eines Arzneimittels verwendet werden können.

In einer bevorzugten Ausführungsform werden Anthracen-Derivate gemäß der allgemeinen Formel (II) wie eben dargestellt bereitgestellt, worin:
Substituent **R27** unabhängig ausgewählt ist aus der Gruppe bestehend aus: "3,4-Dimethoxy-phenyl; 3,4-Dihydroxy-phenyl; 4-Methoxy-thiophen-2-yl, Thiomorpholin-4-yl; 4-Methoxy-phenyl; 4-(4-Methoxy-phenyl)-piperazin-1-yl, 2-Hydroxy-3,4-dimethoxy-phenyl";
die zur Herstellung eines Arzneimittels verwendet werden können.

In einer bevorzugten Ausführungsform werden Anthracen-Derivat-Verbindungen **1 bis 8** zur Herstellung eines Arzneimittels bereitgestellt.

Die erfindungsgemäßen Verbindungen können im Rahmen dieser Erfindung zur Behandlung oder Prophylaxe von durch die Inhibition der Tubulin-Polymerisation und/oder von durch die Inhibition Microtubuli-basierten Motorproteinen behandelbaren physiologischen und/oder pathophysiologischen Zuständen verwendet werden.

In einer bevorzugten Ausführungsform sind die Microtubuli-basierten Motorproteine ausgewählt aus der Gruppe bestehend aus: "Kinesine, Dyneine, Kinesin-Superfamilie (KIF) Protein, N-1 Kinesine, KIF5A, KIF5B, KIF5C, N-2 Kinesine, Eg5 (KIF11, KSP), BimC (KIF8), N-3 Kinesine, KIF1A, KIF1B, KIF1C, KIF13A, KIF13B, KIF14, KIF16A, KIF16B, N-4 Kinesine, KIF3A, KIF3B, KIF3C, KIF17, N-5 Kinesine, KIF4, KIF4A, KIF4B, KIF21A, KIF21B, N-6 Kinesine, KIF23 (MKLP1, CH01), KIF20A (Rab6-KIF), KIF20B (KIpMPP1), MKLP2, N-7 Kinesine, KIF10 (CENP-E), N-8 Kinesine, KIF18A, KIF18B, KIF22 (Kid), KIF19A, KIF19B, N-9 Kinesine, KIF12, N-10 Kinesine, KIF15 (HkIp2), N-11 Kinesine, KIF24, KIF25 (KNSL3), KIF26A, KIF26B, M-Kinesine, KIF2A (KIF2), KIF2B, KIF2C (MCAK), C-1 Kinesine, KIFC1, C-2 Kinesine, KIFC2, KIFC3, KIF6, KIF7, KIF9" und sind bevorzugt ausgewählt aus der Gruppe bestehend aus: "Eg5 (KIF11, KSP), K1F4, KIF4A, KIF4B, KIF10 (CENP-E), K1F23 (MKLP1, CHO1)".

In einer weiteren bevorzugten Ausführungsform können die erfindungsgemäßen Verbindungen zur Behandlung oder Prophylaxe von malignen Tumoren, benignen Tumoren, soliden/festen Tumoren, Sarkomen, Karzinomen, hyperproliferativen Erkrankungen, Carcinoiden, Ewing-Sarcomen, Kaposi-Sarkomen, Hirntumoren, Tumoren ausgehend vom Hirn und/oder Nervensystem und/oder Hirnhäuten (WO 99/01764), Gliomen, Neuroblastomen, Magenkrebs, Nierenkrebs, Nierenzellkarzinomen, Prostatakrebs, Prostatakarzinomen, Bindegewebstumoren, Weichteilsarkomen, Pankreastumoren, Lebertumoren, Kopftumoren, Halstumoren, Speiseröhrenkrebs, Schilddrüsenkrebs, Osteosarcomen, Retinoblastomen, Thymom, Hodenkrebs, Lungenkrebs, Bronchialkarzinomen, Brustkrebs, Mammakarzinomen, Darmkrebs, Kolorectaltumoren, Kolonkarzinomen, Rektumkarzinomen, gynokologische Tumoren, Ovartumoren/Ovarialtumoren, Gebärmutterkrebs, Gebärmutterhalskrebs, Zervixkarzinomen, Gebärmutterkörperkrebs, Korpuskarzinomen, Endometriumkarzinomen, Harnblasenkrebs, Blasenkrebs, Hautkrebs, Basaliomen, Spinaliomen, Melanomen, intraoculären Melanomen, Leukämien, chronischen Leukämien, akuten Leukämien und/oder Lymphomen verwendet werden. Diese Indikationen stellen auch durch die Inhibition der Tubulin-Polymerisation und/oder von durch die Inhibition Microtubuli-basierten Motorproteinen behandelbare physiologische und/oder pathophysiologische Zustände dar.

In einem weiteren Aspekt der vorliegenden Erfindung wurde die erfinderische Aufgabe überraschender Weise dadurch gelöst, dass die erfindungsgemäßen Verbindungen gemäß der oben dargestellten Aspekte, bevorzugten Ausführungsformen und Verwendungen bereitgestellt werden, zur Verwendung zur Herstellung eines Arzneimittels, wobei das Arzneimittel zusätzlich mindestens eine weitere pharmakologisch aktive Substanz enthält.

In einem weiteren Aspekt der vorliegenden Erfindung wurde die erfinderische Aufgabe überraschender Weise dadurch gelöst, dass die erfindungsgemäßen Verbindungen gemäß der oben dargestellten Aspekte, bevorzugten Ausführungsformen und Verwendungen bereitgestellt werden, zur Verwendung zur Herstellung eines Arzneimittels, wobei das Arzneimittel vor und/oder während und/oder nach der Behandlung mit mindestens einer weiteren pharmakologisch aktiven Substanz verabreicht wird.

In einem weiteren Aspekt der vorliegenden Erfindung wurde die erfinderische Aufgabe überraschender Weise dadurch gelöst, dass die erfindungsgemäßen Verbindungen gemäß der oben dargestellten Aspekte, bevorzugten Ausführungsformen und Verwendungen bereitgestellt werden, zur Verwendung zur Herstellung eines Arzneimittels, wobei das Arzneimittel vor und/oder während und/oder nach der Behandlung mit Strahlentherapie und/oder Chirurgie verabreicht wird.

Die erfindungsgemäßen Verbindungen können dabei im Rahmen dieser Erfindung als Einzelsubstanzen oder in Kombination mit allen bekannten pharmakologisch aktiven Substanzen in einer Kombinationstherapie wie dargestellt verabreicht werden.

In einer bevorzugten Ausführungsform werden die erfindungsgemäßen Verbindungen bereitgestellt zu den oben dargestellten Verwendungen, wobei die weitere pharmakologisch aktive Substanz ausgewählt ist aus der Gruppe bestehend aus: "DNA Topoisomerase I und/oder II Inhibitoren, DNA Interkalatoren, alkylierende Agentien, Microtubuli Destabilisatoren, Hormon- und/oder Wachstumsfaktor-Rezeptor-Agonisten und/oder -Antagonisten, Hemmstoffen der Signaltransduktion, Antikörper gegen Wachstumsfaktoren und deren Rezeptoren, Kinase Inhibitoren, Antimetabolite".

In einer bevorzugten Ausführungsform werden die erfindungsgemäßen Verbindungen bereitgestellt zu den oben dargestellten Verwendungen, wobei die weitere pharmakologisch aktive Substanz ausgewählt ist aus der Gruppe bestehend aus: "Actinomycin D, Aminoglutethimid, Asparaginase, Avastin, Azathioprin, BCNU (Carmustine), Bleomycin, Busulfan, Carboplatin, CCNU (Lomustine), Chlorambucil, Cisplatin, Colaspase, Cyclophosphamid, Cytarabin, Dactinomycin, Daunorubicin, Diethylstilbestrol, Doxorubicin (Adriamycin), DTIC (Dacarbacin), Epirubicin, Erbitux, Erythrohydroxynonyladenin, Ethinylestradiol, Etoposide, Fludarabin Phosphate, Fluoxymesteron, Flutamid, Gemcitabin, Gleevec/Glivec, Herceptin, Hexamethylmelamin, Hydroxharnstoff, Hydroxyprogesteron Caproat, Idarubicin, Ifosfamid, Interferon, Iressa, Irinotecan, L-Asparaginase, Leucovorin, Mechlorethamin, Medroxyprogesteron Acetat, Megestrol Acetat, Melphalan, Mesna, Methotrexat, Mitomycin C, Mitotan, Mitoxantrone, N-Phosphonoacetyl-L-aspartat (PALA), Oxaliplatin, Pentostatin, Plicamycin, Prednisolon, Prednison, Procarbazin, Raloxifen, Rapamycin, Semustin, Sorafenib, Streptozocin, Tamoxifen, Tarceva, Taxotere, Teniposide, Testosteron Propionat, Thioguanin, Thiotepa, Topotecan, Trimethylmelamin, Uridine, Vinblastin, Vincristin, Vindesin, Vinorelbin, 2', 2'-Difluorodeoxycytidin, 5- Fluorodeoxyuridin Monophosphat, 5-Azacytidin Cladribin, 5-Fluorodeoxyuridin, 5-Fluorouarcil (5-FU), 6-Mercaptopurin".

Die erfindungsgemäßen Arzneimittel können als flüssige, halbfeste und feste Arzneiformen verabreicht werden. Dies erfolgt in der jeweils geeigneten Weise in Form von Aerosolen, Pulver, Puder und Streupuder, Tabletten, Dragees, Emulsionen, Schäume. Lösungen, Suspensionen, Gele, Salben, Pasten, Pillen, Pastillen, Kapseln oder Suppositorien.

Die erfindungsgemäßen Arzneimittel können in einer geeigneten Darreichungsform auf die Haut, epicutan als Lösung, Suspension, Emulsion, Schaum, Salbe, Paste oder Pflaster; über die Mund- und Zungenschleimhaut, buccal, lingual oder sublingual als Tablette, Pastille, Dragees, Linctus oder Gurgelwasser; über die Magen- und Darmschleimhaut, enteral als Tablette, Dragees, Kapsel, Lösung, Suspension oder Emulsion; über die Rectumschleimhaut, rectal als Suppositorium, Rectalkapsel oder Salbe; über die Nasenschleimhaut, nasal als Tropfen, Salben oder Spray; über das Bronchial- und Alveolarepithel, pulmonal oder per inhalationem als Aerosol oder Inhalat; über die Conjunctiva, conjunctival als Augentropfen, Augensalbe, Augentabletten, Lamellae oder Augenwasser; über die Schleimhäute der Genitalorgane, intravaginal als Vaginalkugeln, Salben und Spülung, intrauterin als Uterus-Pessare; über die ableitenden Harnwege, intraurethral als Spülung, Salbe oder Arzneistäbchen; in eine Arterie, intraarteriell als Injektion; in eine Vene, intravenös als Injektion oder Infusion; in die Haut, intracutan als Injektion oder Implantat; unter die Haut, subcutan als Injektion oder Implantat; in den Muskel, intramusculär als Injektion oder Implantat; in die Bauchhöhle, intraperitoneal als Injektion oder Infusion verabreicht werden.

Die orale Verabreichung kann beispielsweise in fester Form als Tablette, Kapsel, Gelkapsel, Dragee, Granulat oder Pulver, jedoch auch in Form einer trinkbaren Lösung erfolgen. Zur oralen Verabreichung können die neuen, erfindungsgemäßen Verbindungen, wie vorstehend definiert, mit bekannten und gewöhnlich verwendeten, physiologisch verträglichen Hilfs- und Trägerstoffen kombiniert werden, wie z.B. Gummiarabikum, Talkum, Stärke, Zucker wie z.B. Mannit, Methylcellulose, Lactose, Gelatine, oberflächenaktive Mittel, Magnesiumstearat, Cyclodextrine, wässrige oder nicht-wässrige Trägerstoffe, Verdünnungsmittel, Dispergiermittel, Emulgatoren, Schmiermittel, Konservierungsstoffe und Geschmacksstoffe (z.B. etherische Öle). Die erfindungsgemäßen Verbindungen können auch in einer mikropartikulären, z.B. nanopartikulären Zusammensetzung dispergiert sein.

Die nicht orale Verabreichung kann beispielsweise durch intravenöse, subkutane oder intramuskuläre Injektion steriler wässriger oder öliger Lösungen, Suspensionen oder Emulsionen, mittels Implantaten oder durch Salben, Cremes oder Suppositorien erfolgen. Gegebenenfalls kann auch eine Verabreichung als Retardform erfolgen. Implantate können inerte Materialen enthalten, z.B. biologisch abbaubare Polymere oder synthetische Silicone wie z.B. Silicongummi. Eine intravaginale Verabreichung kann z.B. mittels Vaginalringen erfolgen. Eine intrauterine Verabreichung kann z.B. mittels Diaphragmen oder anderer geeigneter intrauteriner Vorrichtungen erfolgen. Darüber hinaus ist auch eine transdermale Verabreichung, insbesondere mittels einer dazu geeigneten Formulierung und/oder geeigneter Mittel wie z.B. Pflaster, vorgesehen.

Wie bereits vorstehend erläutert, können die neuen, erfindungsgemäßen Verbindungen auch mit weiteren pharmazeutisch aktiven Wirkstoffen kombiniert werden. Im Rahmen einer Kombinationstherapie können die einzelnen wirksamen Bestandteile gleichzeitig oder getrennt verabreicht werden, und zwar entweder auf demselben Wege (z.B. oral) oder auf getrennten Wegen (z.B. oral und als Injektion). Sie können in gleichen oder unterschiedlichen Mengen in einer Einheitsdosis vorliegen bzw. verabreicht werden. Es kann auch ein bestimmtes Dosierungsregime angewendet werden, sofern dies zweckmäßig erscheint. Auf diese Weise können auch mehrere der neuen, erfindungsgemäßen Verbindungen miteinander kombiniert werden.

Die Dosierung kann je nach Art der Indikation, der Schwere der Erkrankung, der Art der Verabreichung, dem Alter, Geschlecht, Körpergewicht und der Sensitivität des zu behandelnden Subjekts in einem breiten Rahmen variieren. Es entspricht den Fähigkeiten eines Fachmanns, eine "pharmakologisch wirksame Menge" der kombinierten pharmazeutischen Zusammensetzung zu bestimmen. Die Verabreichung kann in einer einzigen Dosis oder mehreren getrennten Dosierungen erfolgen.

Eine geeignete Einheitsdosis ist z.B. 0,001 mg bis 100 mg des Wirkstoffs, d.h. mindestens einer erfindungsgemäßen Verbindung und gegebenenfalls einer weiteren pharmazeutisch aktiven Substanz, pro kg Körpergewicht eines Patienten.

In einem weiteren Aspekt der vorliegenden Erfindung sind demnach auch pharmazeutische Zusammensetzungen umfassend eine pharmakologisch aktive Menge mindestens einer erfindungsgemäßen Verbindung, bevorzugt **Verbindung 1, 2, 3, 4, 5, 6**, **7,** und/oder **Verbindung 8,** sowie gegebenenfalls pharmazeutisch verträgliche Träger- und/oder Hilfsstoffe, von der vorliegenden Erfindung erfasst.

Bevorzugte und besonders bevorzugte pharmazeutische Zusammensetzungen sind jene, die mindestens eine der vorstehend genannten bevorzugten erfindungsgemäßen Verbindungen umfassen. In pharmazeutischen Zusammensetzungen gemäß der vorliegenden Erfindung können neben mindestens einer erfindungsgemäßen Verbindung, wie vorstehend definiert, auch noch mindestens eine weitere pharmazeutisch aktive Substanz vorliegen, wie vorstehend bereits näher aufgeführt.

In den erfindungsgemäßen pharmazeutischen Zusammensetzungen liegt mindestens eine der neuen, erfindungsgemäßen Verbindungen, wie vorstehend definiert, in einer pharmakologischen aktiven Menge, vorzugsweise in einer Einheitsdosis, z.B. der vorstehend genannten Einheitsdosis, vor, und zwar vorzugsweise in einer Verabreichungsform, die eine orale Verabreichung ermöglicht.

Bezüglich der erfindungsgemäßen Verbindungen umfassende pharmazeutische Zusammensetzungen sowie bezüglich der Verwendung der erfindungsgemäßen Verbindungen als Arzneimittel sei hinsichtlich Verwendungs- und Verabreichungsmöglichkeiten auf das bereits im Zusammenhang mit der Verwendung der neuen, erfindungsgemäßen Verbindungen selbst, Gesagte verwiesen.

In einem weiteren Aspekt der vorliegenden Erfindung wurde die erfinderische Aufgabe überraschender Weise gelöst durch die Bereitstellung eines Kits umfassend eine pharmakologisch aktive Menge mindestens einer bevorzugten erfindungsgemäßen Verbindung, wie oben dargestellt, und eine pharmakologisch aktive Menge mindestens einer weiteren pharmakologisch aktiven Substanz wie vorstehend definiert.

### Chemische Synthese:

Die Verbindungen der allgemeinen Formeln (I) und (II) sind beispielsweise gemäß dem folgenden Schema 1 erhältlich. Die sich in Formel (II) von Formel (I) unterscheidenden Reste R18 bis R34 sind analog zu den Resten R1 bis R17 der Formel (I) zu betrachten.

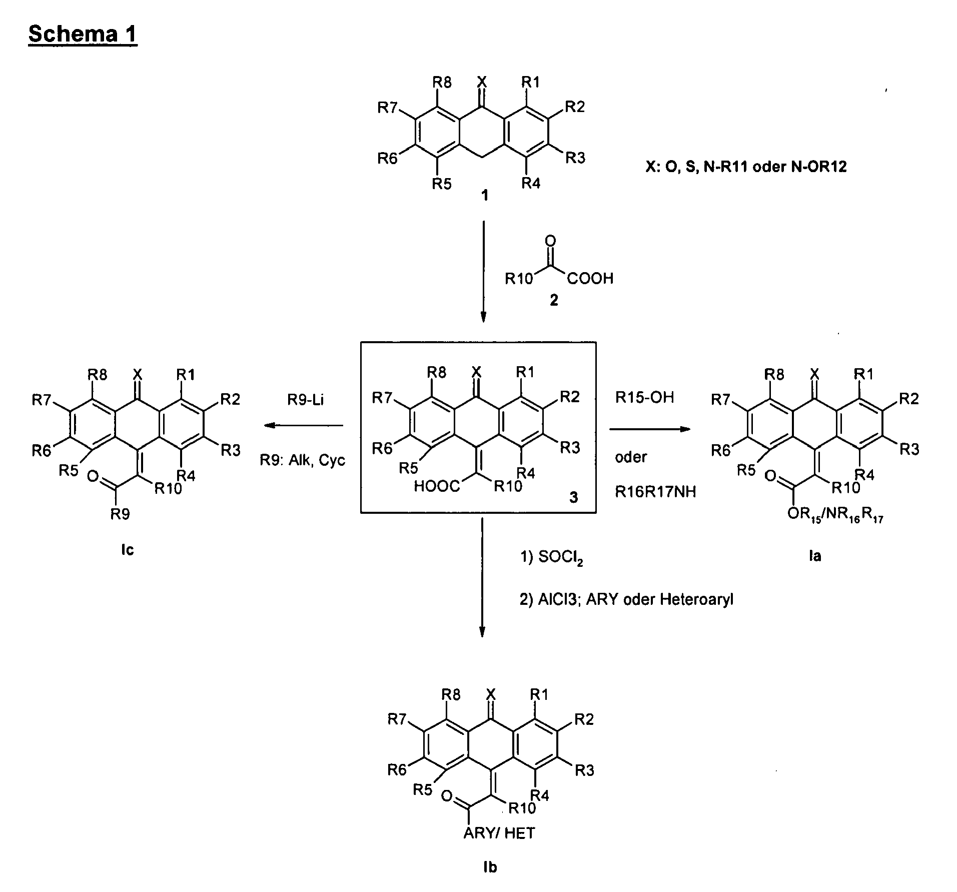

Die Ausgangsverbindungen **1** und **2** sind entweder im Handel erhältlich oder können nach an sich bekannten Verfahrensweisen hergestellt werden. Die Verbindung **3** stellt eine wertvolle Zwischenverbindung für die Herstellung der erfindungsgemäßen Verbindungen der Formeln **(I)** und **(II)** dar.

Die gegebenenfalls zu verwendenden Lösungs- und Hilfsmittel und anzuwendenden Reaktionsparameter wie Reaktionstemperatur und -dauer sind dem Fachmann aufgrund seines Fachwissens bekannt.

Die Benennung der erfindungsgemäßen Verbindungen, insbesondere der Verbindungen **1 bis 8**, erfolgte mit der AutoNom 2000 - Software (ISIS ^{™}/ Draw 2.5; MDL).

Die Inhalte aller zitierten Patentschriften und Referenzen werden hiermit durch Bezugnahme aufgenommen.

Die Erfindung wird anhand der nachfolgenden Beispiele näher erläutert, ohne jedoch auf diese Beispiele beschränkt zu sein.

### Beispiele

### I) Herstellung von erfindungsgemäßen Verbindungen

### Beispiel 1:

### 10-[2-(3,4-Dihydroxy-phenyl)-2-oxo-ethylidene]-10H-anthracen-9-one (Verbindung 1)

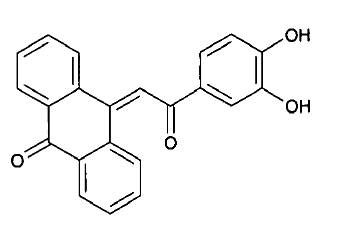

### Stufe 1: Darstellung der 10-Oxo-9(10H)-anthracenyliden-essigsäure

Man suspendierte 10.0 g 9(10*H*)Anthracenon (51.5 mmol) und 9.2 g Glyoxylsäure-Monohydrat (10.0 mmol) in 150 mL Ethanol. Das Gemisch wurde unter Stickstoff zum Sieden erhitzt. Während des Aufheizens gab man 6 Tropfen Piperidin zu, wobei sich das 9(10*H*)Anthracenon auflöste.

Nach 4h war die Umsetzung abgeschlossen. Nach Abkühlung auf Raumtemperatur wurde anschließend der Ansatz auf 400 mL H₂O und 10 mL 6N HCl gegossen und mit Dichlormethan (5x 50 mL) extrahiert. Die vereinigten organischen Phasen wurden mehrfach mit Wasser gewaschen, über Na₂SO₄ getrocknet und im Wasserstrahlvakuum (Rotationsverdampfer) auf ein geringes Volumen eingeengt (30-40 mL).

Die farblose bis leicht gelblich gefärbte 10-Oxo-9(10*H*)-anthracenyliden-essigsäure fiel beim Stehenlassen im Eisbad (1 h) aus. Man saugte ab, presste den Filterkuchen und wusch mit 100 mL kaltem Dichlormethan. Dabei wurde nicht umgesetztes 9(10*H*)Anthracenon herausgelöst. Das Filtrat wurde zur Gewinnung weiterer Säure nochmals eingeengt und wie vorstehend behandelt. Das blass-gelbe Pulver wurde getrocknet (Trockenpistole) und ohne weitere Reinigung für die nächste Stufe 2 verwendet.

Ausbeute 7.60 g (59%) blass-gelber Feststoff

Schmelzpunkt Fp.: 180-182 °C

FTIR: 1684, 1663 cm⁻¹

¹H-NMR (DMSO-d6) δ = 8.15-8.07 (m, 2H), 7.93-7.91 (m, 1 H), 7.77-7.60 (m, 3H), 7.52-7.43 (m, 2H), 7.08 (s, 1H).

### Stufe 2: Darstellung von 10-Oxo-9(10H)-anthracenyliden-essigsäurechlorid

2.0 g 10-Oxo-9(10*H*)-anthracenyliden-essigsäure (7.99 mmol) wurden in 30 ml Thionylchlorid suspendiert. Hierzu gab man 2 Tropfen N,N-Dimethylformamid und erhitzte unter Rückfluss für die Dauer von 2h zum Sieden. Anschließend ließ man auf Raumtemperatur abkühlen und entfernte das Thionylchlorid im Wasserstrahlvakuum. Der Rückstand wurde noch 2x mit jeweils 30 ml n-Hexan versetzt und dieses jeweils im Wasserstrahlvakuum bis zur Trockene entfernt. Das so erhaltene 10-Oxo-9(10*H*)-anthracenyliden-essigsäurechlorid konnte ohne weitere Aufreinigung in Stufe 3 weiterumgesetzt werden.

Ausbeute 2.0 g (93%) gelber Feststoff

Schmelzpunkt Fp.: 115 °C (Zers.)

FTIR: 1757, 1658 cm⁻¹

¹H-NMR (DMSO-d6) δ = 8.15-8.07 (m, 3H), 7.93-7.91 (m, 1H), 7.77-7.60 (m, 4H), 7.08 (s, 1 H).

### Stufe 3a: Synthese von 10-[2-(3,4-Dimethoxy-phenyl)-2-oxo-ethyliden]-9(10H)-anthracenon (Verbindung 6)

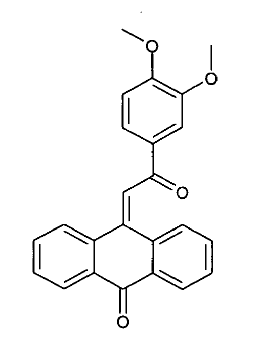

2.15 g (8.0 mmol) 10-Oxo-9(10*H*)-anthracenyliden-essigsäurechlorid wurden unter Kühlung (Eis-Kochsalz-Bad) in 30 mL 1,2-Dichlorethan suspendiert. Anschließend gab man 1.07 g wasserfreies AlCl₃ (8 mmol) in einer Portion zu. Man rührte 10 min im Eisbad und tropfte dann 1.11 g (8.00 mmol) 1-,2- Dimethoxybenzol, gelöst in 5 mL 1,2-Dichlorethan, zu. Das Gemisch färbte sich dunkel und das 10-[2-(3,4-Dimethoxyphenyl)-2-oxo-ethyliden]-9(10*H*)-anthracenon löste sich. Es wurde unter Eiskühlung gerührt (DC-Kontrolle, SiO₂/DCM). Nach beendeter Umsetzung (1-2 h) goss man auf 300 mL Wasser und 50 mL 6N HCl, rührte 10 min und extrahiert dann mit Dichlormethan (4x 50 mL). Die vereinigten organischen Phasen wurden mehrfach mit Wasser gewaschen und über Natriumsulfat getrocknet. Man entfernte das Lösungsmittel im Wasserstrahlvakuum und reinigte den Rückstand mittels Säulenchromathographie an Kieselgel (EE/PE 7:3), wobei 0.97 g an 10-[2-(3,4-Dimethoxy-phenyl)-2-oxo-ethyliden]-9(10*H*)-anthracenon **(Verbindung 6)** als gelber Feststoff resultierten.

Ausbeute: 0.97 g (33% d. Th.) gelber Feststoff

Schmelzpunkt Fp.: 160 °C

FTIR: 1655, 1639 cm⁻¹

¹H-NMR (DMSO-d6) δ= 8.32 (d, 1 H); 8.20 (d, 1 H); 8.14 (d, 1 H); 7.81 (t, 1 H); 7.70 (s, 1 H); 7.69 (t, 1 H); 7.60 (d, 1 H); 7.55 (t, 1 H); 7.46 - 752 (m, 3H); 7.02 (d, 1 H); 3.82 (s, 3H); 3.79 (s, 3H)ppm.

### Stufe 3b: 10-[2-(3,4-Dihydroxy-phenyl)-2-oxo-ethylidene]-10H-anthracen-9-one (Verbindung 1)

Eine Lösung von 342mg (1.0 mmol) 10-[2-(3,4-Dimethoxy-phenyl)-2-oxo-ethyliden]-9(10H)-anthracenon **(Verbindung 6)** in 10 mL getrockneten Dichlormethan wurde bei - 70 °C (Aceton/Trockeneis) zu einer Lösung der 5-7fachen molaren Menge BBr₃ in 20 mL DCM getropft (N₂-Atmosphere). Man rührte das Gemisch 24 h, wobei man den Ansatz langsam auf Raumtemperatur erwärmen ließ. Anschließend goss man auf einen Überschuss Wasser und schüttelte kräftig durch. Man extrahierte mit Ethylacetat, trocknete über Na₂SO₄ und engte im Wasserstrahlvakuum ein. Der Rückstand wurde mittels Säulenchromathographie an Kieselgel gereinigt (EE/PE 7:3). Nach dem Einengen der Reinfraktionen kristallisierten 102mg von 10-[2-(3,4-Dihydroxy-phenyl)-2-oxoethylidene]-10H-anthracen-9-one **(Verbindung 1)** durch Zugabe von Hexan aus.

Ausbeute: 102 mg (30% d. Th.) gelbes, amorphes Pulver

Schmelzpunkt Fp.: 217°C

FTIR: 3255 (br), 1634 cm⁻¹

¹H-NMR (d6-DMSO) δ = 8.27 (d, 1H, J = 8.21 Hz), 8.19-8.17 (m, 1H), 8.14-8.11 (m, 1 H), 7.82-7.78 (m, 1 H), 7.68-7.64 (m, 2H), 7.54-7.47 (m, 3H), 7.36-7.33 (m, 2H), 6.78-6.76 (d, 1H);

Folgende Verbindungen der allgemeinen Formeln **(I)** und **(II)** wurden analog zum Syntheseweg in Schema 1 synthetisiert:

### Beispiel 2:

### 10-[2-(4-Methoxy-thiophen-2-yl)-2-oxo-ethylidene]-10H-anthracen-9-one (Verbindung 2)

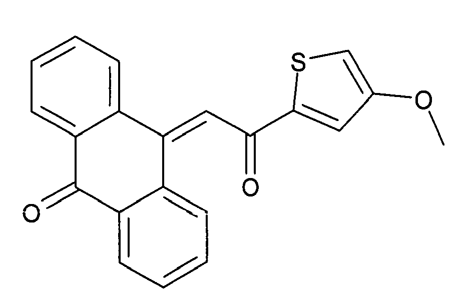

Schmelzpunkt Fp.: 200-201 °C

FTIR: 1648, 1621 cm⁻¹

¹H-NMR (DMSO-d6) δ= 8.18 (d, 1 H); 8.12 - 8.15 (m, 2H); 8.04 (d, 1 H); 7.83 (t, 1 H); 7.65-7.70 (m, 2H); 7.52 - 7.59 (m, 2H); 7.52 (s, 1H); 7.11 (d, 1 H); 3.81 (s, 3H) ppm.

### Beispiel 3:

### 10-(2-Oxo-2-thiomorpholin-4-ylethylidene)-10H-anthracen-9-one (Verbindung 3)

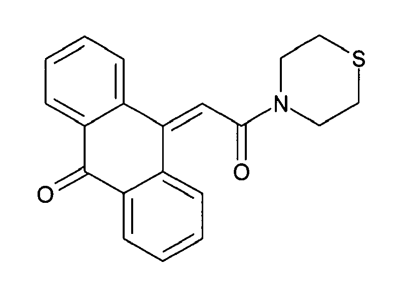

Schmelzpunkt Fp.: 177-178°C

FTIR: 1659, 1628 cm⁻¹

¹H-NMR (DMSO-d6) δ= 8.21 (d, 1 H); 8.17 (d, 1 H); 8.14 (d, 1 H); 7.87 (d, 1 H); 7.79 (t, 1 H); 7.78 (t, 1 H); 7.67 (t, 1 H); 7.64 (t, 1 H); 7.22 (s, 1 H); 3.85 (t, 1 H); 3.51 (t, 1 H); 2.62 (t, 1 H); 2.13 (t, 1 H) ppm.

### Beispiel 4:

### 2-(10-Hydroxy-10H-anthracen-9-yldene)-1-(4-methoxy-phenyl)-ethanone (Verbindung 4)

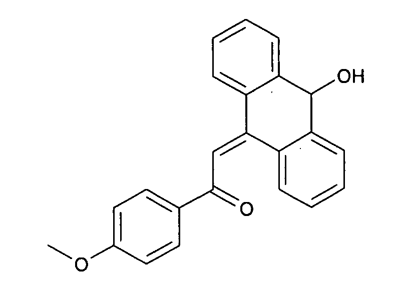

Schmelzpunkt Fp.: 169-172°C

FTIR: 3421, 1638 cm⁻¹

¹H-NMR (DMSO-d6) δ= 7.98(d, 2H); 7.91 (d, 1 H); 7.46 (t, 1 H); 7.41 (t, 1 H); 7.31 (t, 1 H); 7.25 (d, 1 H); 7.12 (s, 1 H); 7.08 (t, 1 H); 6.98 (d, 2H) 6.30 (d, 1 H); 5.54 (d, 1 H); 3.82 (s, 3H) ppm.

### Beispiel 5:

### 10-{2-[4-(4-Methoxy-phenyl)-piperazin-1-yl]-2-oxo-ethylidene}-10H-anthracen-9-on (Verbindung 5)

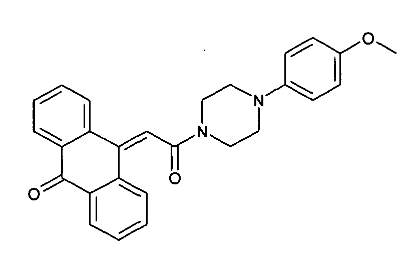

Schmelzpunkt Fp.: 189-190°C

¹H-NMR (DMSO-d6) δ= 8.16 -8.22 (m, 3H); 7.87 (d, 1 H); 7.80 (t, 1 H); 7.73 (t, 1 H); 7.65 (t, 1 H); 7.63 (t, 1 H); 7.25 (s, 1 H); 6.80 (m, 4H); 3.72 (m, 2H); 3.67 (s, 3H); 3.01 (m, 2H); 2.58 (m, 2H) ppm.

### Beispiel 6:

### 10-[2-(3,4-Dimethoxy-phenyl)-2-oxo-ethyliden]-9(10H)-anthracenon (Verbindung 6)

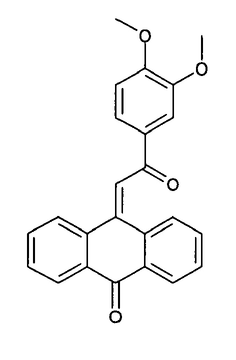

Schmelzpunkt Fp.: 160 °C

FTIR: 1655, 1639 cm⁻¹

¹H-NMR (DMSO-d6) δ= 8.32 (d, 1 H); 8.20 (d, 1 H); 8.14 (d, 1 H); 7.81 (t, 1 H); 7.70 (s, 1 H); 7.69 (t, 1 H); 7.60 (d, 1 H); 7.55 (t, 1 H); 7.46 - 752 (m, 3H); 7.02 (d, 1 H); 3.82 (s, 3H); 3.79 (s, 3H)ppm.

### Beispiel 7:

### 10-[2-(4-Methoxy-phenyl)-2-oxo-ethylidene]-10H-anthracen-9-one (Verbindung 7)

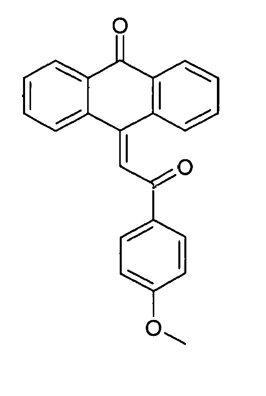

Schmelzpunkt Fp.: 120-122 °C

FTIR 1647, 1591 cm⁻¹

¹H-NMR (DMSO-d6) δ= 8.32 (d, 1 H); 8.20 (d, 1 H); 8.14 (d, 1 H); 7.96 (d, 2H); 7.83 (t, 1 H); 7.72 (s, 1 H); 7.69 (t, 1 H); 7.46- 7.56 (m, 3H); 7.03 (d, 2H); 3.81 (s, 3H) ppm.

### Beispiel 8:

### 10-[2-(2-Hydroxy-3,4-dimethoxy-phenyl)-2-oxo-ethylidene]-10H-anthracen-9-one (Verbindung 8)

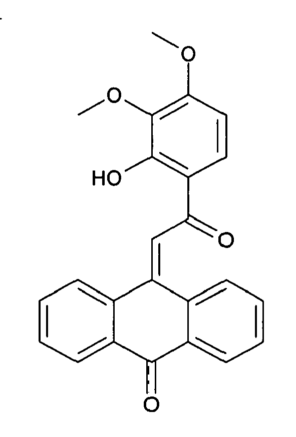

Schmelzpunkt Fp.: 176-178 °C

FTIR 3030, 1655, 1615 cm⁻¹

¹H-NMR (DMSO-d6) δ= 8.12 (d, 1 H); 8.00 (d, 1 H); 7.83 (d, 1 H); 7.71 (t, 1 H); 7.55 (t, 1 H); 7.48 (t, 1 H); 7.38-7.44 (m, 2H); 7.16 (d, 1H); 6.70 (d, 1 H); 5.22 (s, 1 H); 5.17 (s, 1 H); 3.80 (s, 3H), 3.53 (s, 3H)ppm.

### II) Inhibierung der Polymerisation von Tubulin

a) Die erfindungsgemäßen **Verbindungen 1** und **7** wurden in einem in-vitro Test auf Hemmung der Polymerisation von Rindertubulin getestet (D.M. Bollag et al. Cancer Res. 1995, 55: 2325-2333). In diesem Test wird durch Zyklen von Polymerisation und Depolymerisation aufgereinigtes Tubulin eingesetzt, welches durch Zugabe von GTP und Erwärmung zur Polymerisation gebracht wird.
In **Tabelle 1** sind die EC₅₀-Werte der Polymerisationshemmung von Tubulin mit 30% assoziierten Proteinen (MAPs) angegeben.

**Tabelle 1**

| **Verbindung** | **EC50 [µg/mL]** |
|---|---|
| **1** | 7.15/8.02 |
| **7** | 1.38/1.50 |

b) Die **Verbindungen 1, 2, 4** und **7** wurden in einem in-vitro Test auf Hemmung der Polymerisation von Schweinehirntubulin getestet. In diesem Test wird durch Zyklen von Polymerisation und Depolymerisation aufgereinigtes Tubulin eingesetzt (Shelanski et al. Proc. Natl. Acad. Sci. U.S.A. 1973, 70, 765-768 modifiziert nach Vater et al. Acta Histochem. Suppl. 1986, 33, 123-129), welches durch Zugabe von GTP und Erwärmung auf 37°C zur Polymerisation gebracht wird. Das eingesetzte Protein enthält neben dem Tubulin ca. 15% assoziierter Proteine (MAPs). Die Messwerte wurden turbidimetrisch nach Gaskin et al. J. Mol. Biol. 89 (1974) 737-755 erhoben.

In **Tabelle 2** ist der EC₅₀ Wert der Polymerisationshemmung angegeben.

**Tabelle 2**

| **Verbindung** | **EC50 [µg/mL]** |
|---|---|
| **1** | 3.0 |
| **2** | 2.3 |
| **4** | 1.2 |
| **7** | 0.5 |

### III) Inhibition von Kinesin Eg5

Die **Verbindung 1** wurde in einem in-vitro Test auf Hemmung der Eg5-Aktivität untersucht [Kodama, T. et al. J. Biochem. 99: 1465-1472 (1986)].

Dabei kamen die rekombinanten Kinesine Eg5₁₃₋₄₃₇ (Tabelle 3) und Eg5₁₋₄₀₆ (Tabelle 4) zum Einsatz.

In den **Tabellen 3** und **4** sind die EC₅₀ Werte der Eg5-Hemmung für die erfinderische **Verbindung 1** angegeben.

**Tabelle 3**

| **Verbindung** | EC50 **[µg/mL]** |
|---|---|
| **1** | 2.37/ 2.48/ 1.87/ 2.02 |

**Tabelle 4**

| **Verbindung** | **EC50 [µg/mL]** |
|---|---|
| **1** | 12.7 |

### IV) Inhibition von Kinesin KIF5A

Die **Verbindung 1** wurde in einem in-vitro Test auf Hemmung der KIF5A ATPase-Aktivität untersucht. Dabei kam rekombinantes Volllängen KIF5A (Niclas J, Navone F, Hom-Booher N, Vale RD Neuron 1994, 12:1059-1072) zum Einsatz.

Die Aktivität der Mikrotubulus-aktivierbaren ATPase wurden mittels end-point Assay bestimmt. Zur Tubulin-Isolierung und Herstellung der Mikrotubuli s. Zitate im Beispiel II) b).

In **Tabelle 5** ist der EC₅₀ Wert der ATPase- Hemmung von KIF5A durch die erfinderische Verbindung **1** angegeben.

**Tabelle 5**

| **Verbindung** | **EC50 [µg/mL]** |
|---|---|
| **1** | 21.2 |

### V) Antiproliferative Wirkung an verschiedenen Tumorzellinien

Die erfindungsgemäßen **Verbindungen 1, 2, 4, 6** und **7** wurden in einem Proliferationstest an etablierten Tumorzelllinien auf ihre anti-proliferative Aktivität hin untersucht (D.A. Scuderio et al. Cancer Res. 1988, 48: 4827-4833).

Der verwendete Test bestimmt die zelluläre Dehydrogenase-Aktivität und ermöglicht eine Bestimmung der Zellvitalität und indirekt der Zellzahl.

Bei den verwendeten Zelllinien handelt es sich um die humane Cervixkarzinom Zelllinie KB/HeLa (ATCC CCL17), die ovariale Adenokarzinomzelllinie SKOV-3 (ATCC HTB77), die humane Glioblastom Zelllinie SF-268 (NCI 503138) und die Lungenkarzinom Zelllinie NCI-H460 (NCI 503473). Des Weiteren wurde zur Untersuchung der Zellzyklus-spezifischen Wirkung der Substanz ein RKOp27 Zellsystem verwendet (M. Schmidt et al. Oncogene 2000, 19(20): 2423-9).

Bei RKO handelt es sich um eine humane Kolonkarzinomlinie, in der der Zellzyklusinhibitor p27^{kip1} mittels des Ecdyson Expressionssystems induziert zur Expression gebracht und zu einem Zellzyklusarrest spezifisch in G2 geführt werden kann.

Eine unspezifisch wirkende Substanz hemmt die Proliferation unabhängig davon, ob die RKO Zelle in G1 oder G2 arretiert ist oder nicht. Zellzyklus-spezifische Substanzen wie beispielsweise Tubulininhibitoren sind hingegen nur dann zytotoxisch, wenn Zellen nicht arretiert sind und der Zellzyklus durchlaufen wird.

In **Tabelle 6** ist die zytotoxische bzw. wachstumshemmende Aktivität der beschriebenen Verbindungen mit / ohne Expression von p27 ^{kip1} gezeigt. Die getesteten Verbindungen zeigten im induzierten Zustand von p27^{kip1} keine zytotoxische Aktivität. Die Ergebnisse zeigen eine sehr potente Hemmung der Proliferation ausgewählter Tumorzelllinien durch die erfindungsgemäßen Verbindungen.

**Tabelle 6**

| **Verbindung** | **KB/HeLa EC50 [µg/mL]** | **NCI-H460 EC50 [µg/mL]** | **SF-268 EC50 [µg/mL]** | **SK-OV-3 EC50 [µg/mL]** | **RKOP27 EC50 [µg/mL]** | **RKOP27**^{ind} **EC50 [µg/mL]** |
|---|---|---|---|---|---|---|
| **1** | 0.091 | 0.146 | 0.076 | 0.053 | 0.083 | >3.16 |
| **2** | 0.248 | 0.931 | 0.331 | 0.416 | 0.802 | >3.16 |
| **4** | 0.181 | 0.203 | 0.227 | 0.171 | 0.108 | >3.16 |
| **6** | 0.458 | 0.290 | n.d. | n.d. | 0.240 | >3.16 |
| **7** | 0.020 | 0.025 | 0.020 | 0.020 | 0.027 | >3.16 |

### VI) Antiproliferative Wirkung an verschiedenen multi-drug resistenten (MDR) Tumorzelllinien

Zur weiteren Charakterisierung wurden die erfindungsgemäßen **Verbindungen 6** und **7** gegenüber multi-drug resistenten (MDR) Zelllinien im Vergleich zu den nichtresistenten Wildtypzelllinien untersucht.

Bei den untersuchten Zelllinien handelt es sich um die murine L1210 (ATCC CCL219), die akute myeloische Leukaemie Zelllinie LT12 (De Vries et al.; University Rotterdam) und um die resistenten Linien L1210/VCR (Asta Medica Laboratory) und LT12/mdr (De Vries et al.; University Rotterdam).

Außerdem wurden die murine P388 Zelllinie (methyl-cholanthrene-induced lymphoid neoplasm) [R. Supino et al.; Inst. Nazionale per lo Studio e las cura die Tumori; Milano)] sowie die Doxorubicin-resistente P388 (NSC-123127) als Testsysteme herangezogen.

In der **Tabelle 7** sind die entsprechenden XTT-Daten dargestellt.

**Tabelle 7**

| **Verbindung** | **LT12 IC50 [µg/mL]** | **LT12mdr IC50 [µg/mL]** | **L1210 IC50 [µg/mL]** | **L1210VCR IC50 [µg/mL]** | **P388 IC50 [µg/mL]** | **P388ADR IC50 [µg/mL]** |
|---|---|---|---|---|---|---|
| **6** | 0.10/ | 0.10/ | 0.32/ | 0.26/ | 0.35/ | 0.25/ |
| | 0.17 | 0.23 | 0.35 | 0.54 | 0.39 | 0.49 |
| **7** | 0.015/ | 0.015/ | 0.016/ | 0.017/ | 0.014/ | 0.016/ |
| | 0.019 | 0.020 | 0.017 | 0.018 | 0.015 | 0.018 |

### VII) Zellzyklus-Analyse

Der Zellzyklus umfasst die Entwicklung der Zelle von einer Zellgeneration zur nächsten.

Während der Ruhephase (G0) und präsynthetischen Phase (G1) besitzt die Zelle einen diploiden Chromosomensatz (2c). In der Synthesephase (S) wird die DNA Menge durch Replikation vermehrt. Die S-Phase endet mit Erreichen der prämitotischen Phase (G2M), in der die Zelle einen reduplizierten Chromosomenbestand (4c) und verdoppelten DNA-Gehalt aufweist. In der nachfolgenden, kurzdauernden Mitosephase (M) kommt es zur gleichmäßigen Aufteilung der reduplizierten Chromosomen auf zwei Tochterzellen, die dann jeweils wieder einen diploiden DNA Gehalt zeigen und sich in der G01-Phase befinden, so daß der Zellzyklus neu beginnen kann.

Für die Zellzyklusanalyse wurden KB/HeLa Zellen (ATCC CCL17) mit den Testsubstanzen in unterschiedlichen Konzentrationen (0.1-1000 nM) 24 Stunden bei 37°C behandelt.

Der prozentuale Anteil der in der G2/M Phase des Zellzyklus arretierten Zellen nach Behandlung mit erfindungsgemäßen Verbindungen und ausgewählten Referenzsubstanzen ist in der nachfolgenden **Tabelle 8** dargestellt. Die Ergebnisse wurden mit einer speziellen Analysesoftware (ModFit^{™}) ausgewertet.

**Tabelle 8**

| **Verbindung** | **EC50 [nM] (50% cells in G2/M)** |
|---|---|
| **1** | 0.131/0.303 |
| **paclitaxel** | 26.9 |

## Patentansprüche

1. Anthracen-Derivat gemäß der allgemeinen Formel **(I)** 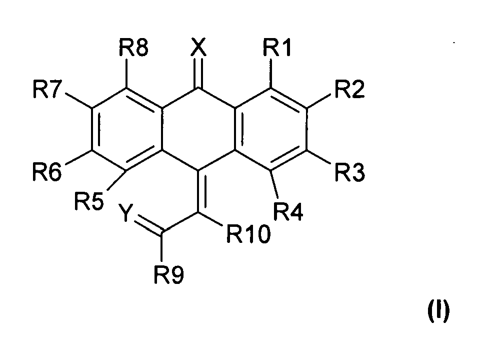 worin:
Substituent **X** unabhängig ausgewählt ist aus der Gruppe bestehend aus: "O, S, NR11, N-OR12, geminal geknüpfter Wasserstoff und Hydroxy";
Substituent Y unabhängig ausgewählt ist aus der Gruppe bestehend aus: "O, S, NR13, N-OR14";
Substituenten **R1, R2, R3, R4, R5, R6, R7, R8** unabhängig voneinander unabhängig ausgewählt sind aus der Gruppe bestehend aus: "Wasserstoff, unsubstituiertes oder substituiertes Alkyl, unsubstituiertes oder substituiertes Cycloalkyl, unsubstituiertes oder substituiertes Cycloalkyl-alkyl, unsubstituiertes oder substituiertes Heterocyclyl, unsubstituiertes oder substituiertes Heterocyclyl-alkyl, unsubstituiertes oder substituiertes Aryl, unsubstituiertes oder substituiertes Aryl-alkyl, unsubstituiertes oder substituiertes Heteroaryl, unsubstituiertes oder substituiertes Heteroaryl-alkyl, Amino, Mono-Alkylamino, Di-Alkylamino, Halogen, -F, -Cl, -Br, -I, mit ein oder mehreren Fluoratomen substituiertes Alkyl, Trifluormethyl, Cyano, geradkettiges oder verzweigtes Cyano-alkyl, Carbonyl, Carboxyl, -COOH, Alkoxycarbonyl, Carboxy-alkyl, Alkoxycarbonyl-alkyl, Hydroxy, Alkoxy, Aryl-alkoxy, Benzyloxy, Heteroaryl-alkoxy, Alkoxycarbonylamino, Alkoxycarbonylamino-alkyl";
Substituent **R9** unabhängig ausgewählt ist aus der Gruppe bestehend aus: "unsubstituiertes oder substituiertes Alkyl, unsubstituiertes oder substituiertes Cycloalkyl, unsubstituiertes oder substituiertes Cycloalkyl-alkyl, unsubstituiertes oder substituiertes Heterocyclyl, unsubstituiertes oder substituiertes Heterocyclyl-alkyl, unsubstituiertes oder substituiertes Aryl, unsubstituiertes oder substituiertes Aryl-alkyl, unsubstituiertes oder substituiertes Heteroaryl, unsubstituiertes oder substituiertes Heteroaryl-alkyl, -OR15, -NR16R17";
Substituent **R10** unabhängig ausgewählt ist aus der Gruppe bestehend aus: "Wasserstoff, unsubstituiertes oder substituiertes Alkyl, unsubstituiertes oder substituiertes Aryl, unsubstituiertes oder substituiertes Aryl-alkyl, unsubstituiertes oder substituiertes Heteroaryl, unsubstituiertes oder substituiertes Heteroaryl-alkyl";
Substituenten **R11, R12, R13, R14, R15, R16, R17** unabhängig voneinander unabhängig ausgewählt sind aus der Gruppe bestehend aus: "Wasserstoff, unsubstituiertes oder substituiertes Alkyl, unsubstituiertes oder substituiertes Cycloalkyl, unsubstituiertes oder substituiertes Cycloalkyl-alkyl, unsubstituiertes oder substituiertes Heterocyclyl, unsubstituiertes oder substituiertes Heterocyclyl-alkyl, unsubstituiertes oder substituiertes Aryl, unsubstituiertes oder substituiertes Aryl-alkyl, unsubstituiertes oder substituiertes Heteroaryl, unsubstituiertes oder substituiertes Heteroaryl-alkyl";
mit der Maßgabe, dass die folgenden, unter die allgemeine Formel (I) fallenden Verbindungen ausgeschlossen sind:
(i) Verbindung mit der Chemical Abstract Service (CAS) Registry Nr. 262378-52-9 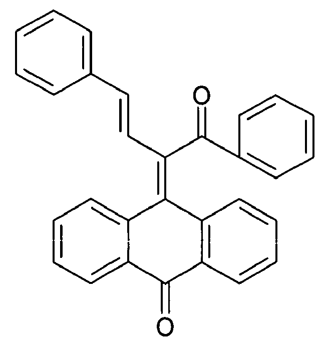
(ii) Verbindung mit der Chemical Abstract Service (CAS) Registry Nr. 121747-08-8 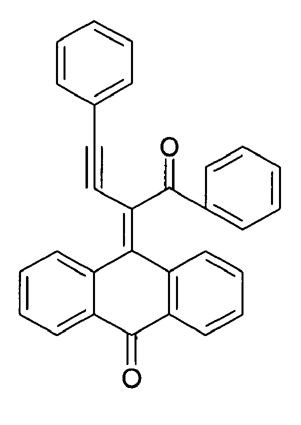
(iii) Verbindung mit der Chemical Abstract Service (CAS) Registry Nr. 63370-49-0 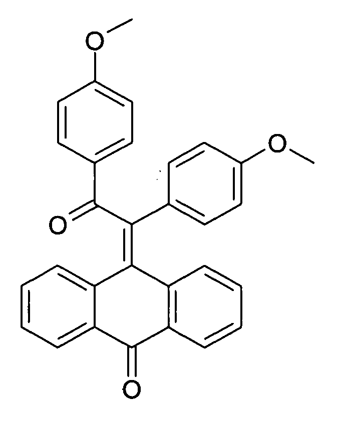
(iv) Verbindung mit der Chemical Abstract Service (CAS) Registry Nr. 19661-62-2 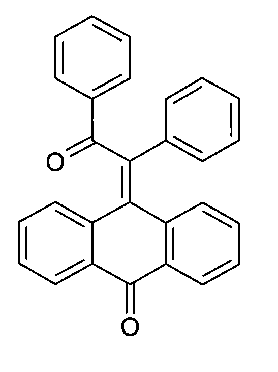
(v) Verbindung mit der Chemical Abstract Service (CAS) Registry Nr. 17665-75-7 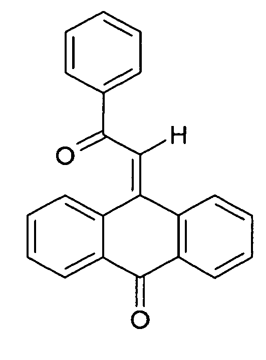
(vi) Verbindung mit der Chemical Abstract Service (CAS) Registry Nr. 83498-19-5

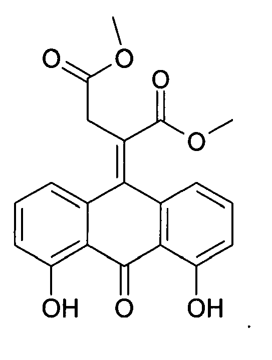

2. Anthracen-Derivat gemäß der allgemeinen Formel **(I)** gemäß Anspruch 1, worin:
Substituent X unabhängig ausgewählt ist aus der Gruppe bestehend aus: "O, geminal geknüpfter Wasserstoff und Hydroxy";
Substituent **Y** unabhängig "O" ist;
Substituenten **R1, R2, R3, R4, R5, R6, R7, R8** unabhängig "Wasserstoff' sind;
Substituent **R9** unabhängig ausgewählt ist aus der Gruppe bestehend aus: "unsubstituiertes oder substituiertes Heterocyclyl, unsubstituiertes oder substituiertes Aryl, unsubstituiertes oder substituiertes Heteroaryl";
Substituent **R10** unabhängig "Wasserstoff" ist.

3. Anthracen-Derivat gemäß der allgemeinen Formel **(I)** gemäß einem der Ansprüche 1 bis 2, worin:
Substituent **R9** unabhängig ausgewählt ist aus der Gruppe bestehend aus: "3,4-Dimethoxy-phenyl; 3,4-Dihydroxy-phenyl; 4-Methoxy-thiophen-2-yl, Thiomorpholin-4-yl; 4-Methoxy-phenyl; 4-(4-Methoxy-phenyl)-piperazin-1-yl, 2-Hydroxy-3,4-dimethoxy-phenyl".

4. Anthracen-Derivat ausgewählt aus der Gruppe bestehend aus:
10-[2-(3,4-Dihydroxy-phenyl)-2-oxo-ethylidene]-10H-anthracen-9-one **(Verbindung 1)**: 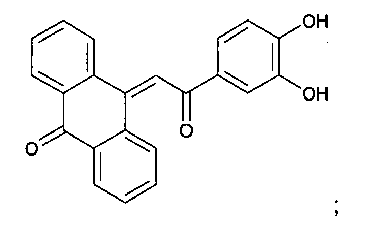
10-[2-(4-Methoxy-thiophen-2-yl)-2-oxo-ethylidene]-10H-anthracen-9-one **(Verbindung 2):** 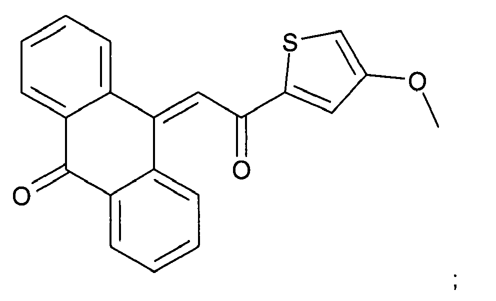
10-(2-Oxo-2-thiomorpholin-4-ylethylidene)-10H-anthracen-9-one **(Verbindung 3):** 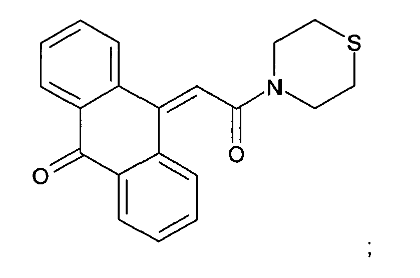
2-(10-Hydroxy-10H-anthracen-9-ylidene)-1-(4-methoxy-phenyl)-ethanone **(Verbindung 4):** 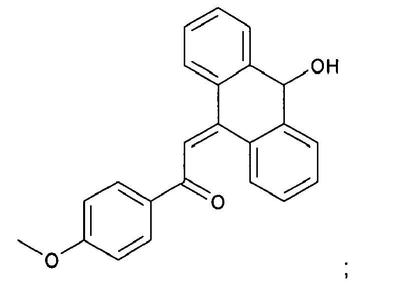
10-{2-[4-(4-Methoxy-phenyl)-piperazin-1-yl]-2-oxo-ethylidene}-10H-anthracen-9-one **(Verbindung 5):** 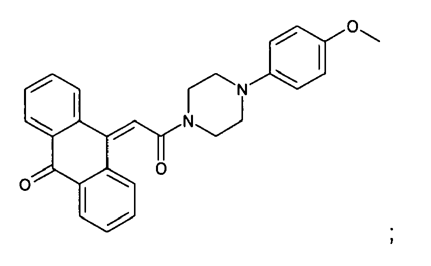
10-[2-(3,4-Dimethoxy-phenyl)-2-oxo-ethyliden]-9(10*H*)-anthracenon **(Verbindung 6):** 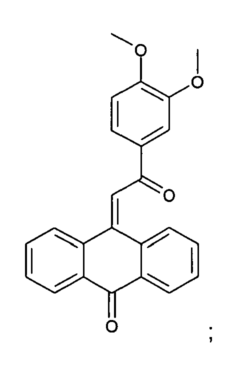
10-[2-(4-Methoxy-phenyl)-2-oxo-ethylidene]-10H-anthracen-9-one **(Verbindung 7):** 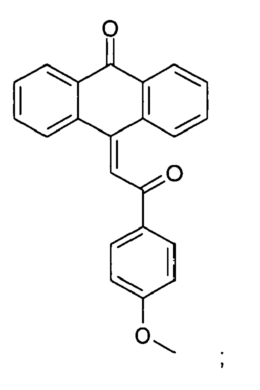
10-[2-(2-Hydroxy-3,4-dimethoxy-phenyl)-2-oxo-ethylidene]-10H-anthracen-9-one **(Verbindung 8)**

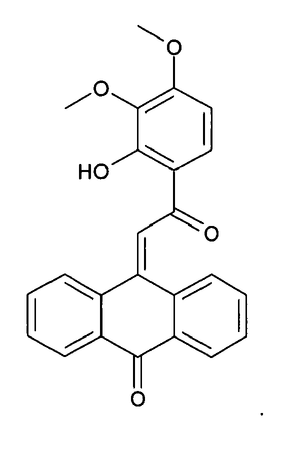

5. Verfahren zur Herstellung von Anthracen-Derivaten gemäß einem der Ansprüche 1 bis 4.

6. Pharmazeutische Zusammensetzung enthaltend eine pharmazeutisch aktive Menge mindestens einer Verbindung gemäß einem der Ansprüche 1 bis 4.

7. Pharmazeutische Zusammensetzung gemäß Anspruch 6, wobei die mindestens eine Verbindung in einer Einheitsdosis von 0.001 mg bis 100 mg pro kg Körpergewicht des Patienten vorliegt.

8. Pharmazeutische Zusammensetzung gemäß einem der Ansprüche 6 bis 7, wobei die Zusammensetzung zusätzlich pharmazeutisch akzeptable Träger- und/oder Hilfsstoffe enthält.

9. Pharmazeutische Zusammensetzung gemäß einem der Ansprüche 6 bis 8, wobei die Zusammensetzung zusätzlich mindestens eine weitere pharmakologisch aktive Substanz enthält.

10. Pharmazeutische Zusammensetzung gemäß Anspruch 9, wobei die weitere pharmakologisch aktive Substanz ausgewählt ist aus der Gruppe bestehend aus: "DNA Topoisomerase I und/oder II Inhibitoren, DNA Interkalatoren, alkylierende Agentien, Microtubuli Destabilisatoren, Hormon- und/oder Wachstumsfaktor-Rezeptor-Agonisten und/oder -Antagonisten, Hemmstoffen der Signaltransduktion, Antikörper gegen Wachstumsfaktoren und deren Rezeptoren, Kinase Inhibitoren, Antimetabolite".

11. Pharmazeutische Zusammensetzung gemäß Anspruch 10, wobei die weitere pharmakologisch aktive Substanz ausgewählt ist aus der Gruppe bestehend aus: "Actinomycin D, Aminoglutethimid, Asparaginase, Avastin, Azathioprin, BCNU (Carmustine), Bleomycin, Busulfan, Carboplatin, CCNU (Lomustine), Chlorambucil, Cisplatin, Colaspase, Cyclophosphamid, Cytarabin, Dactinomycin, Daunorubicin, Diethylstilbestrol, Doxorubicin (Adriamycin), DTIC (Dacarbacin), Epirubicin, Erbitux, Erythrohydroxynonyladenin, Ethinylestradiol, Etoposide, Fludarabin Phosphate, Fluoxymesteron, Flutamid, Gemcitabin, Gleevec/Glivec, Herceptin, Hexamethylmelamin, Hydroxharnstoff, Hydroxyprogesteron Caproat, Idarubicin, Ifosfamid, Interferon, Iressa, Irinotecan, L-Asparaginase, Leucovorin, Mechlorethamin, Medroxyprogesteron Acetat, Megestrol Acetat, Melphalan, Mesna, Methotrexat, Mitomycin C, Mitotan, Mitoxantrone, N-Phosphonoacetyl-L-aspartat (PALA), Oxaliplatin, Pentostatin, Plicamycin, Prednisolon, Prednison, Procarbazin, Raloxifen, Rapamycin, Semustin, Sorafenib, Streptozocin, Tamoxifen, Tarceva, Taxotere, Teniposide, Testosteron Propionat, Thioguanin, Thiotepa, Topotecan, Trimethylmelamin, Uridine, Vinblastin, Vincristin, Vindesin, Vinorelbin, 2', 2'-Difluorodeoxycytidin, 5- Fluorodeoxyuridin Monophosphat, 5-Azacytidin Cladribin, 5-Fluorodeoxyuridin, 5-Fluorouarcil (5-FU), 6-Mercaptopurin".

12. Verwendung eines Anthracen-Derivats gemäß der allgemeinen Formel **(II)** 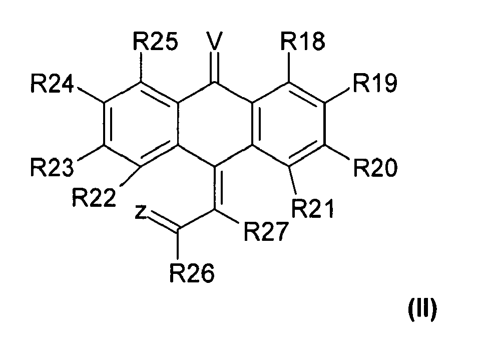 worin:
Substituent **V** unabhängig ausgewählt ist aus der Gruppe bestehend aus: "O, S, NR28, N-OR29, geminal geknüpfter Wasserstoff und Hydroxy;
Substituent **Z** unabhängig ausgewählt ist aus der Gruppe bestehend aus: "O, S, NR30, N-OR31";
Substituenten **R18, R19, R20, R21, R22, R23, R24, R25** unabhängig voneinander unabhängig ausgewählt sind aus der Gruppe bestehend aus: "Wasserstoff, unsubstituiertes oder substituiertes Alkyl, unsubstituiertes oder substituiertes Cycloalkyl, unsubstituiertes oder substituiertes Cycloalkyl-alkyl, unsubstituiertes oder substituiertes Heterocyclyl, unsubstituiertes oder substituiertes Heterocyclyl-alkyl, unsubstituiertes oder substituiertes Aryl, unsubstituiertes oder substituiertes Aryl-alkyl, unsubstituiertes oder substituiertes Heteroaryl, unsubstituiertes oder substituiertes Heteroaryl-alkyl, Amino, Mono-Alkylamino, Di-Alkylamino, Halogen, -F, -Cl, -Br, - I, mit ein oder mehreren Fluoratomen substituiertes Alkyl, Trifluormethyl, Cyano, geradkettiges oder verzweigtes Cyano-alkyl, Carbonyl, Carboxyl, -COOH, Alkoxycarbonyl, Carboxy-alkyl, Alkoxycarbonyl-alkyl, Hydroxy, Alkoxy, Aryl-alkoxy, Benzyloxy, Heteroaryl-alkoxy, Alkoxycarbonylamino, Alkoxycarbonylamino-alkyl";
Substituent **R26** unabhängig ausgewählt ist aus der Gruppe bestehend aus: "unsubstituiertes oder substituiertes Alkyl, unsubstituiertes oder substituiertes Cycloalkyl, unsubstituiertes oder substituiertes Cycloalkyl-alkyl, unsubstituiertes oder substituiertes Heterocyclyl, unsubstituiertes oder substituiertes Heterocyclyl-alkyl, unsubstituiertes oder substituiertes Aryl, unsubstituiertes oder substituiertes Aryl-alkyl, unsubstituiertes oder substituiertes Heteroaryl, unsubstituiertes oder substituiertes Heteroaryl-alkyl, -OR32, -NR33R34";
Substituent **R27** unabhängig ausgewählt ist aus der Gruppe bestehend aus: "Wasserstoff, unsubstituiertes oder substituiertes Alkyl, unsubstituiertes oder substituiertes Aryl, unsubstituiertes oder substituiertes Aryl-alkyl, unsubstituiertes oder substituiertes Heteroaryl, unsubstituiertes oder substituiertes Heteroaryl-alkyl";
Substituenten **R28, R29, R30, R31, R32, R33, R34** unabhängig voneinander unabhängig ausgewählt sind aus der Gruppe bestehend aus: "Wasserstoff, unsubstituiertes oder substituiertes Alkyl, unsubstituiertes oder substituiertes Cycloalkyl, unsubstituiertes oder substituiertes Cycloalkyl-alkyl, unsubstituiertes oder substituiertes Heterocyclyl, unsubstituiertes oder substituiertes Heterocyclyl-alkyl, unsubstituiertes oder substituiertes Aryl, unsubstituiertes oder substituiertes Aryl-alkyl, unsubstituiertes oder substituiertes Heteroaryl, unsubstituiertes oder substituiertes Heteroaryl-alkyl";
zur Herstellung eines Arzneimittels.

13. Verwendung eines Anthracen-Derivats gemäß der allgemeinen Formel **(II)** gemäß Anspruch 12, worin:
Substituent **V** unabhängig ausgewählt ist aus der Gruppe bestehend aus: "O, geminal geknüpfter Wasserstoff und Hydroxy";
Substituent **Z** unabhängig "O" ist;
Substituenten **R18, R19, R20, R21, R22, R23, R24, R25** unabhängig "Wasserstoff" sind;
Substituent **R26** unabhängig ausgewählt ist aus der Gruppe bestehend aus: "unsubstituiertes oder substituiertes Heterocyclyl, unsubstituiertes oder substituiertes Aryl, unsubstituiertes oder substituiertes Heteroaryl";
Substituent **R27** unabhängig "Wasserstoff" ist;
zur Herstellung eines Arzneimittels.

14. Verwendung eines Anthracen-Derivats gemäß der allgemeinen Formel **(II)** gemäß einem der Ansprüche 12 bis 13, worin:
Substituent **R27** unabhängig ausgewählt ist aus der Gruppe bestehend aus: "3,4-Dimethoxy-phenyl; 3,4-Dihydroxy-phenyl; 4-Methoxy-thiophen-2-yl, Thiomorpholin-4-yl; 4-Methoxy-phenyl; 4-(4-Methoxy-phenyl)-piperazin-1-yl, 2-Hydroxy-3,4-dimethoxy-phenyl";
zur Herstellung eines Arzneimittels.

15. Verwendung eines Anthracen-Derivats gemäß Anspruch 4 zur Herstellung eines Arzneimittels.

16. Verwendung einer Verbindung gemäß einem der Ansprüche 4, 12 bis 14 zur Herstellung eines Arzneimittels zur Behandlung oder Prophylaxe von durch die Inhibition der Tubulin-Polymerisation und/oder von durch die Inhibition Microtubuli-basierten Motorproteinen behandelbaren physiologischen und/oder pathophysiologischen Zuständen.

17. Verwendung gemäß Anspruch 16, wobei die Microtubuli-basierten Motorproteine ausgewählt sind aus der Gruppe bestehend aus: "Kinesine, Dyneine, Kinesin-Superfamilie (KIF) Protein, N-1 Kinesine, KIF5A, KIF5B, KIF5C, N-2 Kinesine, Eg5 (KIF11, KSP), BimC (KIF8), N-3 Kinesine, KIF1A, KIF1B, KIF1C, KIF13A, KIF13B, KIF14, KIF16A, KIF16B, N-4 Kinesine, KIF3A, KIF3B, KIF3C, KIF17, N-5 Kinesine, KIF4, KIF4A, KIF4B, KIF21A, KIF21B, N-6 Kinesine, KIF23 (MKLP1, CHO1) KIF20A (Rab6-KIF), KIF20B (KIpMPP1), MKLP2, N-7 Kinesine, KIF10 (CENP-E), N-8 Kinesine, KIF18A, KIF18B, KIF22 (Kid), KIF19A, KIF19B, N-9 Kinesine, KIF12, N-10 Kinesine, KIF15 (Hklp2), N-11 Kinesine, KIF24, KIF25 (KNSL3), KIF26A, KIF26B, M-Kinesine, KIF2A (KIF2), KIF2B, KIF2C (MCAK), C-1 Kinesine, KIFC1, C-2 Kinesine, KIFC2, KIFC3, KIF6, KIF7, KIF9" und bevorzugt ausgewählt aus der Gruppe bestehend aus: "Eg5 (KIF11, KSP), KIF4, KIF4A, KIF4B, KIF10 (CENP-E), KIF23 (MKLP1, CH01)" sind.

18. Verwendung gemäß einem der Ansprüche 12 bis 17 zur Behandlung oder Prophylaxe von malignen Tumoren, benigne Tumoren, soliden/festen Tumoren, Sarkomen, Karzinomen, hyperproliferativen Erkrankungen, Carcinoiden, Ewing-Sarcomen, Kaposi-Sarkomen, Hirntumoren, Tumoren ausgehend vom Hirn und/oder Nervensystem und/oder Hirnhäuten, Gliomen, Neuroblastomen, Magenkrebs, Nierenkrebs, Nierenzellkarzinomen, Prostatakrebs, Prostatakarzinomen, Bindegewebstumoren, Weichteilsarkomen, Pankreastumoren, Lebertumoren, Kopftumoren, Halstumoren, Speiseröhrenkrebs, Schilddrüsenkrebs, Osteosarcomen, Retinoblastomen, Thymom, Hodenkrebs, Lungenkrebs, Bronchialkarzinomen, Brustkrebs, Mammakarzinomen, Darmkrebs, Kolorectaltumoren, Kolonkarzinomen, Rektumkarzinomen, gynokologische Tumoren, Ovartumoren/Ovarialtumoren, Gebärmutterkrebs, Gebärmutterhalskrebs, Zervixkarzinomen, Gebärmutterkörperkrebs, Korpuskarzinomen, Endometriumkarzinomen, Harnblasenkrebs, Blasenkrebs, Hautkrebs, Basaliomen, Spinaliomen, Melanomen, intraoculären Melanomen, Leukämien, chronischen Leukämien, akuten Leukämien und/oder Lymphomen".

19. Verwendung gemäß einem der Ansprüche 12 bis 18, wobei das Arzneimittel zusätzlich mindestens eine weitere pharmakologisch aktive Substanz enthält.

20. Verwendung gemäß einem der Ansprüche 12 bis 19, wobei das Arzneimittel vor und/oder während und/oder nach der Behandlung mit mindestens einer weiteren pharmakologisch aktiven Substanz verabreicht wird.

21. Verwendung gemäß einem der Ansprüche 19 bis 20, wobei die weitere pharmakologisch aktive Substanz ausgewählt ist aus der Gruppe bestehend aus: "DNA Topoisomerase I und/oder II Inhibitoren, DNA Interkalatoren, alkylierende Agentien, Microtubuli Destabilisatoren, Hormon- und/oder Wachstumsfaktor-Rezeptor-Agonisten und/oder -Antagonisten, Hemmstoffen der Signaltransduktion, Antikörper gegen Wachstumsfaktoren und deren Rezeptoren, Kinase Inhibitoren, Antimetabotite".

22. Verwendung gemäß Anspruch 21, wobei die weitere pharmakologisch aktive Substanz ausgewählt ist aus der Gruppe bestehend aus: "Actinomycin D, Aminoglutethimid, Asparaginase, Avastin, Azathioprin, BCNU (Carmustine), Bleomycin, Busulfan, Carboplatin, CCNU (Lomustine), Chlorambucil, Cisplatin, Colaspase, Cyclophosphamid, Cytarabin, Dactinomycin, Daunorubicin, Diethylstilbestrol, Doxorubicin (Adriamycin), DTIC (Dacarbacin), Epirubicin, Erbitux, Erythrohydroxynonyladenin, Ethinylestradiol, Etoposide, Fludarabin Phosphate, Fluoxymesteron, Flutamid, Gemcitabin, Gleevec/Glivec, Herceptin, Hexamethylmelamin, Hydroxharnstoff, Hydroxyprogesteron Caproat, Idarubicin, Ifosfamid, Interferon, Iressa, Irinotecan, L-Asparaginase, Leucovorin, Mechlorethamin, Medroxyprogesteron Acetat, Megestrol Acetat, Melphalan, Mesna, Methotrexat, Mitomycin C, Mitotan, Mitoxantrone, N-Phosphonoacetyl-L-aspartat (PALA), Oxaliplatin, Pentostatin, Plicamycin, Prednisolon; Prednison, Procarbazin, Raloxifen, Rapamycin, Semustin, Sorafenib, Streptozocin, Tamoxifen, Tarceva, Taxotere, Teniposide, Testosteron Propionat, Thioguanin, Thiotepa, Topotecan, Trimethylmelamin, Uridine, Vinblastin, Vincristin, Vindesin, Vinorelbin, 2', 2'-Difluorodeoxycytidin, 5- Fluorodeoxyuridin Monophosphat, 5-Azacytidin Cladribin, 5-Fluorodeoxyuridin, 5-Fluorouarcil (5-FU), 6-Mercaptopurin".

23. Verwendung gemäß einem der Ansprüche 12 bis 22, wobei das Arzneimittel vor und/oder während und/oder nach der Behandlung mit Strahlentherapie und/oder Chirurgie verabreicht wird.

24. Kit umfassend eine pharmakologisch aktive Menge mindestens einer Verbindung gemäß einem der Ansprüche 4, 12 bis 14 und eine pharmakologisch aktive Menge mindestens einer weiteren pharmakologisch aktiven Substanz gemäß einem der Ansprüche 9 bis 11.
